# EUROPEAN PATENT SPECIFICATION

(11) **EP 0 393 575 B1**
(45) Date of publication and mention of the grant of the patent: **16.03.1994**
(21) Application number: 90107246.2
(22) Date of filing: 17.04.1990
(51) Int. Cl.: A61K 45/06, A61K 31/785

(54) **Neoplasia treatment compositions containing antineoplastic agent and side-effect reducing protective agent**
Zusammensetzungen zur Behandlung von Neoplasia mit Gehalt an neoplasmushemmendem Mittel und Nebenwirkungen reduzierendem Schutzmittel
Composition pour le traitement de néoplasia contenant un agent antinéoplastique et un agent protecteur réduisant les effets secondaires

(30) Priority: 17.04.1989 US 339503
(43) Date of publication of application: 24.10.1990
(73) Proprietor: G.D. Searle & Co., Chicago Illinois 60680 (US)
(72) Inventor: Bach, Ardalan, Coconut Grove Florida 33133 (US); Shanahan, William R.Jr, Ledyard, Connecticut 06339 (US)
(74) Representative: Beil, Hans Chr., Dr.

(56) References cited:
- GB-A- 2 040 951
- CHEMICAL ABSTRACTS, vol. 103, no. 12, 23rd September 1985, page 323, abstract no. 92828x, Columbus, Ohio, US;
- CHEMICAL ABSTRACTS, vol. 103, no. 16, 21st October 1985, page 341, abstract no. 128863w, Columbus, Ohio, US; S. MIYAZAKI et al.: "Pharmaceutical application of biomedical polymers. Part XIV. Antitumor effect of implanted ethylene-vinyl alcohol copolymer matrixes containing anticancer agents on Ehrlich ascites carcinoma and P388 leukemia in mice", & CHEM. PHARM. BULL. 1985, 33(6), 2490-8
- CHEMICAL ABSTRACTS, vol. 107, no. 5, 3rd August 1987, page 30, abstract no. 32707e, Columbus, Ohio, US; F. ZUNINO et al.: "Increased therapeutic efficacy and reduced toxicity of doxorubicin linked to pyran copolymer via the side chain of the drug", & CANCER TREAT. REP. 1987, 71(4), 367-73

## Description

This invention is in the field of neoplastic therapy and relates specifically to therapeutic agents and methods for treatment of neoplastic diseases.

There are many chemotherapeutic agents available for treatment of neoplastic diseases. The design or selection of a chemotherapeutic drug for antineoplastic treatment must take into account many factors, depending on the tumor type, the physical condition of the patient and the progression of tumor growth in the patient.

One set of factors to consider in selecting a chemotherapeutic agent relates to the heterogeneity of tumors. A single tumor type may contain many sub-populations of neoplastic cells with variations in karyotype, morphology, immunogenicity, rate of growth, capacity to metastasize and responsiveness to a antineoplastic agent or a combination of two or more antineoplastic agents [P. Calabresi et al, Biochem. Pharmacol., 28, 1933-1941 (1979)].

Another factor to consider in identifying an effective chemotherapeutic agent is the degree of selectivity of the agent's cytotoxic effect for the tumor cell over normal host cells. One goal of effective chemotherapeutic therapy is to obtain maximum differentiation or a high selectivity ratio in cell killing effects of a cytotoxic agent for tumor cells over normal cells. Factors which affect selectivity include dose rate, that is, the amounts of a drug administered over a period of time, and the intervals of time between cycles of chemotherapy dosing. An important benefit of a high selectivity ratio is reduction of side effects associated with chemotherapy, such as nausea, vomiting, hair loss, organ and bone degeneration, premature aging or death.

In view of the great number of factors which determine effective chemotherapeutic treatment of neoplasia, it has been difficult to identify a single chemical agent which is effective in clinical treatment to produce either significant remissions or to cure patients with cancer [V. T. DeVita et al, N. Engl. J. Med., 288, 998-1006 (1973)]. For example, an antineoplastic agent proven to be highly effective against many types of tumors is doxorubicin, also known as adriamycin. Doxorubicin, as well as other anthracycline-type drugs such as daunorubicin, exhibit cardiac toxicity side effects which have limited wider use of these antineoplastic agents. In some patients treated with doxorubicin, effective cancer remission has been achieved but such remission was accompanied by severe and, in some cases irreversible, cardiomyopathies. Other cancer patients, known to have existing cardiac problems and who might have been successfully treated with doxorubicin or other anthracycline-type drugs, have been denied such treatment because of the cardiotoxicity side effects. Doxorubicin and daunorubicin are also known to interfere with normal cell growth in bone marrow and the gastrointestinal mucosa [V. T. DeVita et al, Cancer: Principles & Practice of Oncology, 2nd Edn., J. P. Lippencott Co., Philadelphia, Pa., p. 313 (1985)].

More effective tumor remission and cures have been accomplished by use of combination chemotherapy, that is, the use of two or more antineoplastic agents in combination. Such combination therapy can provide maximum tumor cytotoxic effect within the toxicity range tolerable by the patient, and can provide cytotoxic effects over a larger number of tumor cell sub-populations, as well as to contain or suppress the development of new resistant cell lines within tumor growth.

There are large numbers of antineoplastic agents available in commercial use, in clinical evaluation and in pre-clinical development, which could be selected for treatment of tumor cell growth by combination drug chemotherapy. Such antineoplastic agents fall into several major categories, namely, antibiotic-type agents, alkylating agents, antimetabolite agents, hormonal agents, immunological agents, interferon-type agents and a category of miscellaneous agents. An example of a compound of this last category is carbetimer which is an antineoplastic agent having significant cytotoxic activity in clonogenic assays [Kisner et al, Proc. ASCO, 2, (1983)] and in nude mice bearing a variety of human tumors [B. Ardalan et al, Cancer Research, 46, (1986)].

Side-effect reduction, without a substantial loss of tumor cytotoxic action, has been accomplished by neoplasia combination chemotherapy. For example, within the antibiotic-type agent category, certain anthracycline-like compounds have been investigated in combination with other antineoplastic agents. Of particular interest is the anthracycline-like compound doxorubicin which is effective in treatment of acute leukemia, malignant lymphomas and certain solid tumors. Doxorubicin and the bispiperazinedione compound ICRF 187, in combination, have been shown to provide reduced cardiotoxic side effects along with maintenance of tumor cytotoxic action.

There continues a need for chemotherapy combinations which will provide cytotoxic action in a wider variety of tumor cell types, or in the more resistant tumor types, along with a reduction in harmful or undesirable side effects.

In Chemical Abstracts (1985), 103, No.92828x and Chemical Abstracts (1987), Vol. 107, No. 32707e the preparation of neoplasm inhibitors (especially of doxorubicin) bound to pyran-copolymers is disclosed. Hereby the copolymer and the active agent are covalently bound and are consequently administered simultaneously.
The references, however, are silent on any cytoprotective effect of the copolymer.

Chemical Abstracts (1985), 103, No. 128863w describes several biomedical pyran-polymers acting as matrices for the anticancer agents. Thereby the polymer is used as a vehicle for controlled or sustained release of the active compound. It is, however, nowhere disclosed or suggested that the copolymer is acting as a cytoprotective polymer which itself could be used as a therapeutically effective agent.

Treatment of neoplastic disease is provided by a combination therapy of therapeutically-effective amounts of a cytoprotective copolymer and one or more directly-acting antineoplasitc agents. The phrase "cytoprotective copolymer" is intended to embrace polymeric materials which exhibit a normal-cell-protecting effect in the presence of a cytotoxic agent, namely, an antineoplastic agent having activity in killing neoplastic cells as well as activity in killing normal host cells. Effective cytoprotective copolymers are provided by half-amide:half-imide copolymers of which carbetimer is a specific example.

The phrase "directly-acting antineoplastic agent" is intended to embrace antineoplastic agents which exert antineoplastic effects directly on the tumor cell, e.g., by cytostatic or cytocidal effects, and not indirectly through mechanisms such as biological response modification. A suitable antineoplastic agent for use in this combination therapy may be selected from antimetabolite-type agents, alkylating-type agents, antibiotic-type agents and other non-classifiable cytotoxically-effective antineoplastic agents. The phrase "combination therapy", as used herein, is intended to embrace administration of the cytoprotective copolymer and one or more antineoplastic agents in a sequential manner and also to embrace co-administration of the cytoprotective copolymer and the one or more antineoplastic agents in a simultaneous manner. The phrase "therapeutically-effective" is used to qualify the amounts of each component used in the combination therapy and is intended to define that amount of each component which, in the combination, achieves the dual goal of effective tumor cytotoxic action and reduction of harmful or undesirable side effects (i.e., a "protective-effective amount" of the cytoprotective copolymer).

Such combination therapy may also include use of the cytoprotective copolymer and one or more antineoplastic agents in conjunction with surgical procedures, such as organ resection, and/or in radiation treatment with a radioprotective agent.

A key advantage provided by any of the combination therapies so far described resides in the host-cell protective effect afforded by the cytoprotective copolymer against the cytotoxic action of the other antineoplastic agent or agents present in the combination therapy. Another advantage of the combination therapy of this invention resides in the antineoplastic activity of the cytoprotective copolymer, such as carbetimer, which may be additive to, or synergistic with, the tumor cytotoxic actions of the other antineoplastic agents in the combination.

Fig. 1 is a graph which demonstrates the ability of carbetimer at 1700 mg/kg dose given intraperitoneally to protect against LD₁₀₀ of adriamycin given intraveneously to BDF₁ mice, with carbetimer administration one-half hour before adriamycin administration.

Fig. 2 is a graph which demonstrates the ability of a carbetimer at 500 mg/kg given intraveneously to protect against sub-acute adriamycin toxicity given intraveneously to BDF₁ mice when each compound is given concurrently.

Fig. 3 is a graph which demonstrates the ability of a carbetimer at 250 mg/kg given intraveneously to protect against sub-acute adriamycin toxicity given intraveneously to BDF₁ mice when each compound is given concurrently.

Fig. 4 is a graph which demonstrates the ability of a carbetimer at 125 mg/kg given intraveneously to protect against sub-acute adriamycin toxicity given intraveneously to BDF₁ mice when each compound is given concurrently.

Fig. 5 is a graph which demonstrates the activity of varying doses of carbetimer given intraperitoneally to inhibit growth of a sub-cutaneously implanted human breast cell carcinoma line.

Fig. 6 is a graph which demonstrates that carbetimer, when added to adriamycin, does not interfere with the antineoplastic activity of adriamycin against a sub-cutaneously implanted tumor line MIA C-51 in rats; rather, carbetimer enhances the antineoplastic activity of adriamycin.

A family of cytoprotective copolymers is provided by half-amide:half-imide copolymers comprised of monomeric units of Formula I:
(a) half-amide, half-carboxylate salt 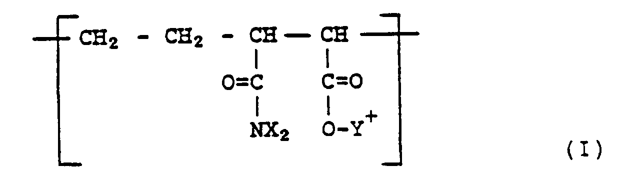 and
(b) imide 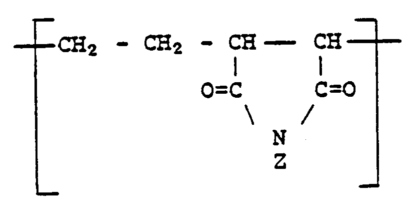
wherein X is selected from hydrido and C₁-C₄ alkyl; wherein Y is selected from hydrido, ammonium and pharmaceutically-acceptable metal cations; and wherein Z is selected from hydrido, C₁-C₄ alkyl, ammonium and pharmaceutically-acceptable metal cations. The (a) and (b) units or groups are distributed along a substantially linear continuous carbon atom molecule. From 5% to 40% of these units should be imide with the balance being principally half-amide, half-carboxylate salt units. These units can be positioned randomly within the chain and/or randomly within the polymer. A small portion (less than 10%) of monoammonium carboxyl or other pharmaceutically-acceptable salt group and/or dicarboxyl group also can be present as may arise from partially reacted or unreacted anhydride during the preparation of these compounds. Of the foregoing derivatized groups, the (a) half-amide, half-carboxylate salt group preferably is half-amide, half-ammonium salt, and the (b) imide group preferably is unsubstituted imide.

A preferred family of cytoprotective half-amide:half-imide copolymers is provided by a copolymer of at least one olefin monomer having from 2 to 4 carbon atoms and at least one α,β-unsaturated polycarboxylic anhydride having from 4 to 6 carbon atoms, having an average molecular weight of from 300 to 1800, and derivatized to obtain both (a) half-amide, half-carboxyl acid groups and (b) imide groups in with said imide groups comprise from 5% by weight to 40% by weight of said derivatized groups, and the N-alkylated derivatives and pharmaceutically-acceptable cationic salt derivatives of said derivatized copolymer, said N-alkylated derivatives having from 1 to 4 carbon atoms in the alkyl substituents.

A more preferred family of cytoprotective half-amide:half-imide copolymers is provided by copolymers comprised of ethylene and maleic anhydride moieties of Formula II:
(a) half-amide, half-ammonium salt 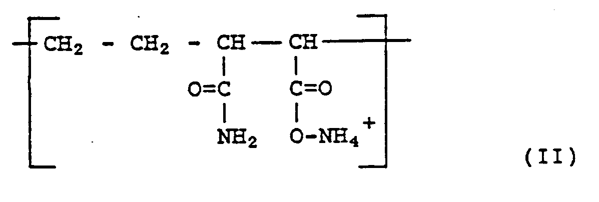 and
(b) unsubstituted imide 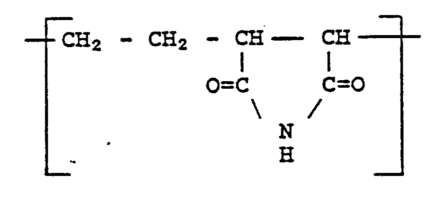
The (a) and (b) units, groups or moieties are distributed along a substantially linear continuous carbon atom molecule. From 5% to 40% of these units are preferably unsubstituted imide with the balance being principally the preferred half-imide, half-ammonium salt units. These units can be positioned randomly within the chain or randomly within the polymer. A small portion (less than 10%) of monoammonium carboxyl or dicarboxyl group can be present as may derive from partially reacted or unreacted anhydride during the preparation of these compounds.

Most preferred is a half-amide:half-imide copolymer of Formula III: 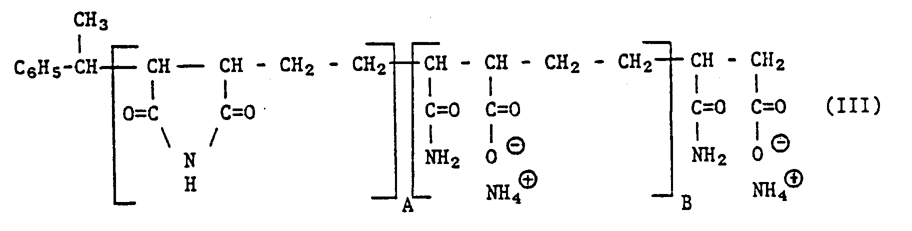
wherein the ratio of A to B is in a range from 1:2 to 1:5.

The cytoprotective half-amide:half-imide copolymer of Formula III is carbetimer, which is a selectively-low molecular weight, water-soluble synthetic polymer also known as by the terms carboxyimamidate, NEP-137, N-137 and POLIMA polymeric material.

Methods for preparation of the copolymers of Formulae I-III are found in U.S. Patent No. 4,255,537,.

A first family of antineoplastic agents which may be used in combination with the cytoprotective copolymer consists of antimetabolite-type antineoplastic agents. Suitable antimetabolite antineoplastic agents may be selected from the group consisting of 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N₁-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT and uricytin.

A second family of antineoplastic agents which may be used in combination with the cytoprotective copolymer consists of alkylating-type antineoplastic agents. Suitable alkylating-type antineoplastic agents may be selected from the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, lomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromus-tine, Tanabe Seiyaku TA-077, tauromustine, temozolomide, teroxirone, tetraplatin and trimelamol.

A third family of antineoplastic agents which may be used in combination with the cytoprotective copolymer consists of antibiotic-type antineoplastic agents. Suitable antibiotic-type antineoplastic agents may be selected from the group consisting of Taiho 4181-A, aclarubicin, actinomycin D, actinoplanone, Erbamont ADR-456, aeroplysinin derivative, Ajinomoto AN-201-II, Ajinomoto AN-3, Nippon soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb, Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindamycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-25024 and zorubicin.

Of this third family of antibiotic-type antineoplastic agents, a class of anthraquinone compounds is preferred. And within this anthraquinone class, anthracycline-type compounds are more preferred. Anthracycline compounds which are particularly preferred are aclarubicin, daunorubicin, ditrisarubicin, doxorubicin, epirubicin, esorubicin, idarubicin, pirarubicin, rodorubicin and zorubicin.

These anthracycline compounds are typically produced by a bacterium of the genus Streptomycetes.

Alternatively, preferred anthracycline compounds are represented by Formula IV: 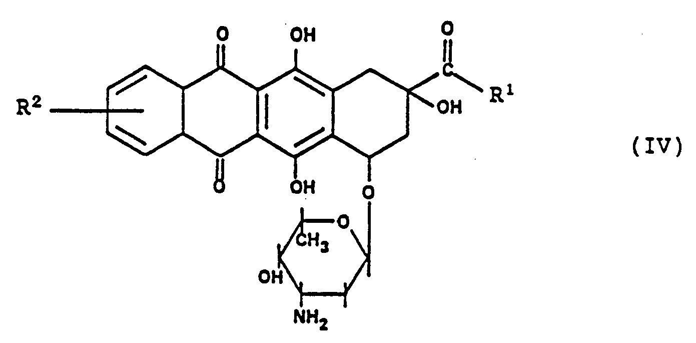
wherein R¹ is selected from hydrido, alkyl, hydroxy, hydroxyalkyl and haloalkyl; and wherein R² is one or more groups selected from alkyl, hydroxy, hydroxyalkyl, alkoxy, alkylthio, halo and carboxylic. Even more preferred are anthracycline compounds wherein R¹ is selected from alkyl and hydroxyalkyl, and wherein R² is alkoxy.

Still more preferred are anthracycline compounds of Formula V: 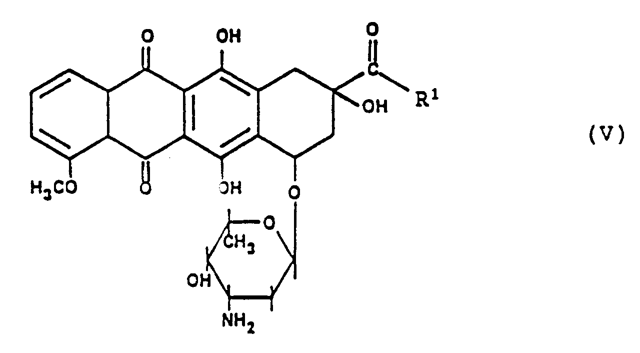
wherein R¹ is selected from alkyl and hydroxyalkyl. More highly preferred are anthracycline compounds wherein R¹ is selected from methyl, ethyl, n-propyl, isopropyl, n-butyl, isobutyl, sec-butyl, tert-butyl, n-pentyl, iso-pentyl, neopentyl, hydroxymethyl, hydroxyethyl, hydroxypropyl, hydroxybutyl and hydroxpentyl.

Most highly preferred of the anthracycline compounds is doxorubicin and daunorubicin, of which doxorubicin is especially preferred.

Certain compounds of the above-mentioned anthracycline-type antineoplastic agents have been shown as effective against a large number of tumor cell lines. For example, doxorubicin is effective in treatment of acute leukemias, malignant lymphomas, Hodgkin's disease, ovarian carcinoma, breast carcinoma, lung small-cell carcinoma, osteogenic carcinoma, Ewing carcinoma, soft-tissue carcinoma, metastatic breast adenocarcinoma, bladder carcinoma, bronchogenic carcinoma, neuroblastoma, metastatic thyroid carcinoma, endometrium carcinoma, testes carcinoma, prostate carcinoma, cervix carcinoma, head and neck carcinoma, gastric carcinoma and plasma-cell myeloma.

A fourth family of antineoplastic agents which may be used in combination with the cytoprotective copolymer consists of a miscellaneous family of antineoplastic agents selected from the group consisting of alpha-carotene, alpha-difluoromethyl-arginine, acitretin, Biotec AD-5, Kyorin AHC-52, alstonine, amonafide, amphethinile, amsacrine, Angiostat, ankinomycin, anti-neoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristo-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, elliprabin, elliptinium acetate, Tsumura EPMTC, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyurea, BTG ICRF-187, ilmofosine, isoglutamine, isotretinoin, Otsuka JI-36, Ramot K-477, Otsuak K-76COONa, Kureha Chemical K-AM, MECT Corp KI-8110, American Cyanamid L-623, leukoregulin, lonidamine, Lundbeck LU-23-112, Lilly LY-186641, NCI (US) MAP, marycin, Merrel Dow MDL-27048, Medco MEDR-340, merbarone, merocyanine derivatives, methylanilinoacridine, Molecular Genetics MGI-136, minactivin, mitonafide, mitoquidone, mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, octreotide, Ono ONO-112, oquizanocine, Akzo Org-10172, pancratistatin, pazelliptine, Warner-Lambert PD-111707, Warner-Lambert PD-115934, Warner-Lambert PD-131141, Pierre Fabre PE-1001, ICRT peptide D, piroxantrone, polyhaematoporphyrin, polypreic acid, Efamol porphyrin, probimane, procarbazine, proglumide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, Topostin, Teijin TT-82, Kyowa Hakko UCN-01, Kyowa Hakko UCN-1028, ukrain, Eastman Kodak USB-006, vinblastine sulfate, vincristine, vindesine, vinestramide, vinorelbine, vintriptol, vinzolidine, withanolides and Yamanouchi YM-534.

Examples of radioprotective agents which may be used in the combination chemotherapy of this invention are AD-5, adchnon, amifostine analogues, detox, dimesna, 1-102, MM-159, N-acylated-dehydroalanines, TGF-α Genentech, tiprotimod, amifostine, WR-151327, FUT-187, ketoprofen transdermal, nabumetone, superoxide dismutase Chiron and superoxide dismutase Enzon.

Methods for preparation of the antineoplastic agents described above may be found in the literature. Methods for preparation of doxorubicin, for example, are described in U.S. Patents No. 3,590,028 and No. 4,012,448,.

Administration of the cytoprotective copolymer and the one or more antineoplastic agents may take place sequentially in separate formulations or may be accomplished by simultaneous administration in a single formulation. Either the cytoprotective copolymer or the antineoplastic agent, or both, may be used in combination with a liposome formulation to deliver the copolymer or agent to the target tumor while protecting more sensitive tissue from the toxic effect of the antineoplastic agent. Administration may be accomplished by oral route, or by intravenous, intramuscular or subcutaneous injections. The formulation may be in the form of a bolus, or in the form of aqueous or non-aqueous isotonic sterile injection solutions or suspensions. These solutions and suspensions may be prepared from sterile powders or granules having one or more pharmaceutically-acceptable carriers or diluents, or a binder such as gelatin or hydroxypropyl-methyl cellulose, together with one or more of a lubricant, preservative, surface-active or dispersing agent.

### BIOLOGICAL EVALUATION

A combination of carbetimer and adriamycin (doxorubicin) was evaluated to study the interaction of carbetimer with doxorubicin in vivo in mouse and rat models.

### Trial I

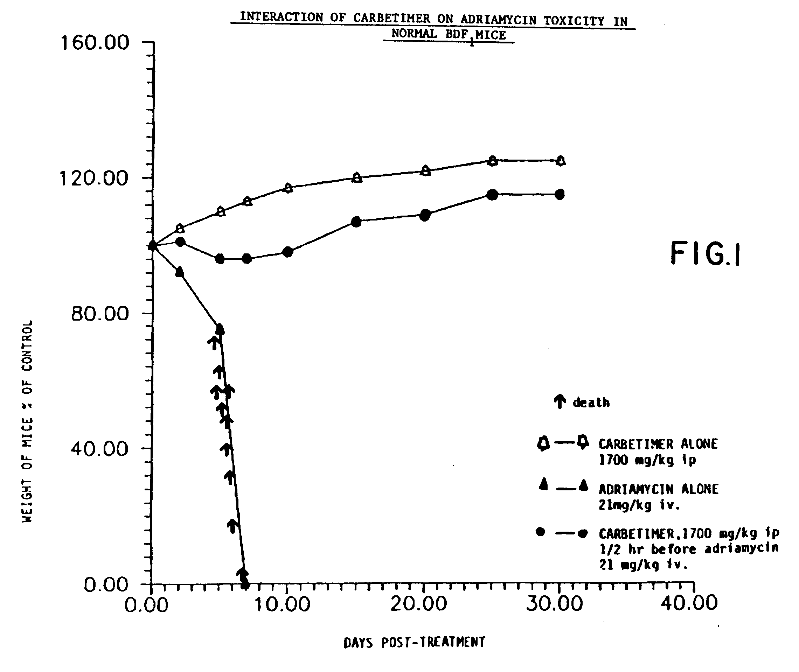

The effect of carbetimer on acute adriamycin toxicity in normal BDF₁ mice was evaluated. Normal BDF₁ mice were treated with carbetimer alone at 1700 mg/kg i.p. with adriamycin alone at 21 mg/kg i.v. or the combination of the two drugs, carbetimer being given half an hour before adriamycin. Animals treated with a lethal dose of adriamycin died within 8 days of the start of the therapy. Animals treated with carbetimer alone gained weight and were all alive at 30 days. In the third group, pre-treatment with carbetimer prevented death which would have otherwise resulted from a lethal dose of adriamycin in all animals. Weight loss was insignificant at the end of the trial. Data are reported in Table I and depicted in Figure 1.

**Table I**

| INTERACTION OF CARBETIMER ON ADRIAMYCIN TOXICITY IN NORMAL BDF₁ MICE | | | |
|---|---|---|---|
| Days Post-treatment | Wt. of Mice (% Control) | | |
| | Carbetimer Alone 1700 mg/kg i.p. | Adriamycin 21 mg/kg i.v. | Carbetimer 1700 mg/kg i.p. + Adriamycin 21 mg/kg i.v. |
| 0 | 100 | 100 | 100 |
| 2 | 105 ± 6 | 92 ± 4 | 101 ± 3 |
| 5 | 108 ± 4 | 79 ± 6 | 95 ± 4 |
| 7 | 112 ± 3 | all dead | 94 ± 3 |
| 10 | 114 ± 4 | - | 96 ± 4 |
| 15 | 116 ± 3 | - | 112 ± 6 |
| 20 | 120 ± 4 | - | 114 ± 8 |
| 25 | 122 ± 3 | - | 115 ± 6 |
| 30 | 123 ± 6 | - | 115 ± 7 |

### Trial II

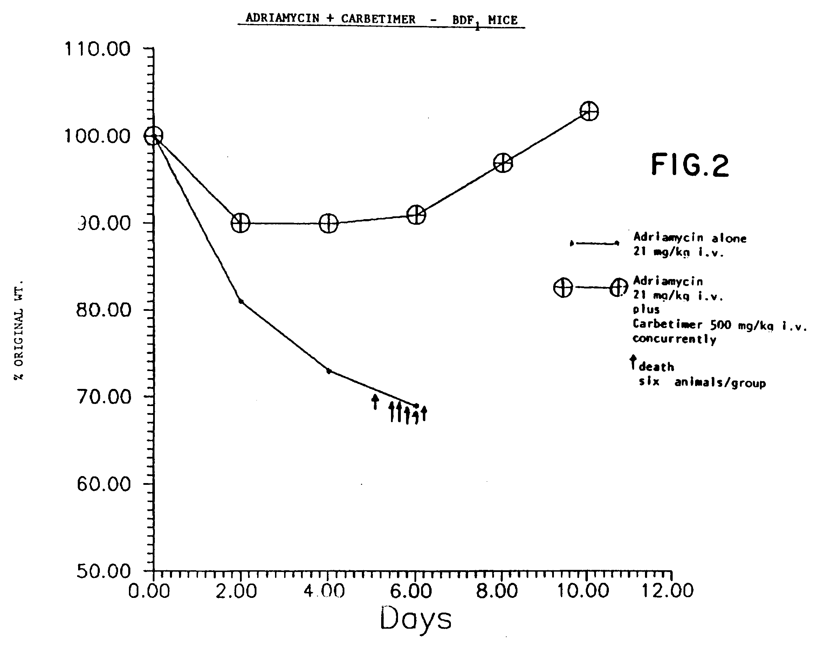

The effect of carbetimer on sub-acute adriamycin toxicity in normal BDF₁ mice was evaluated. Normal BDF₁ mice were treated either with adriamycin alone at 21 mg/kg i.v. or with adriamycin at 21 mg/kg i.v. plus carbetimer 500 mg/kg i.v. given concurrently. The animals treated with a lethal dose of adriamycin died within 8 days of the start of the therapy. In the animals receiving both carbetimer and adriamycin, death was prevented and weight loss was transient and insignificant. Data are reported in Table II in absolute weights of animals and depicted in Figure 2 in terms of percent of original weights of the animals.

**Table II**

| ADRIAMYCIN + CARBETIMER - BDF₁ MICE | | |
|---|---|---|
| Day | Wt. of animals (gm) | |
| | Adriamycin alone 21 mg/kg i.v. | Adriamycin 21 mg/kg i.v. -plus-Carbetimer at 500 mg i.v. |
| 0 | 24 ± 2 | 24 ± 1.5 |
| 2 | 19 ± 0.9 | 21 ± 0.8 |
| 4 | 17 ± 1.2 | 22 ± 0.4 |
| 6 | 16.5 ± 0.8 all dead | 23 ± 1 |
| 8 | - | 25 ± 0.5 |

### Trial III

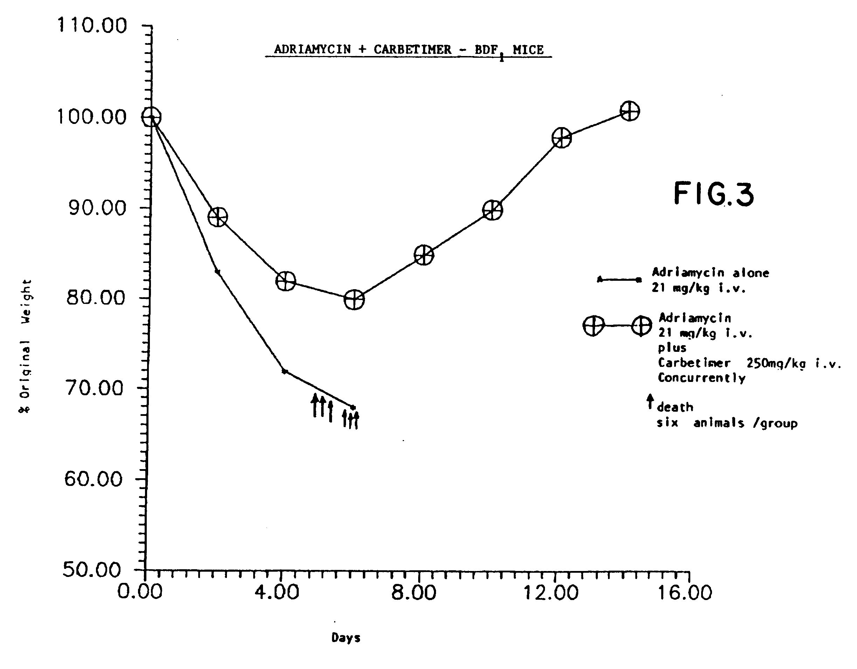

The effect of carbetimer on sub-acute adriamycin toxicity in normal BDF₁ mice was evaluated. Normal BDF₁ mice were treated either with adriamycin alone at 21 mg/kg i.v. or with adriamycin at 21 mg/kg i.v. plus carbetimer 250 mg/kg i.v. given concurrently. The animals treated with a lethal dose of adriamycin died within 8 days of the start of the therapy. In the animals receiving both carbetimer and adriamycin, death was prevented and weight loss was transient. Data are reported in Table III in absolute weights of animals and depicted in Figure 3 in terms of percent of original weights of the animals.

**Table III**

| ADRIAMYCIN + CARBETIMER - BDF₁ MICE | | |
|---|---|---|
| Day | Wt. of animals (gm) | |
| | Adriamycin alone 21 mg/kb i.v. | Adriamycin 21 mg/kg i.v. -plus-Carbetimer at 250 mg i.v. |
| 0 | 22 ± 1 | 22 ± 1 |
| 2 | 18 ± 1.2 | 19 ± 0.8 |
| 4 | 15 ± 0.9 | 18 ± 0.2 |
| 6 | 15 ± 0.4 all dead | 17 ± 0.8 |
| 8 | - | 18 ± 1.2 |
| 10 | - | 19 ± 1.0 |
| 12 | - | 21 ± 0.9 |

### Trial IV

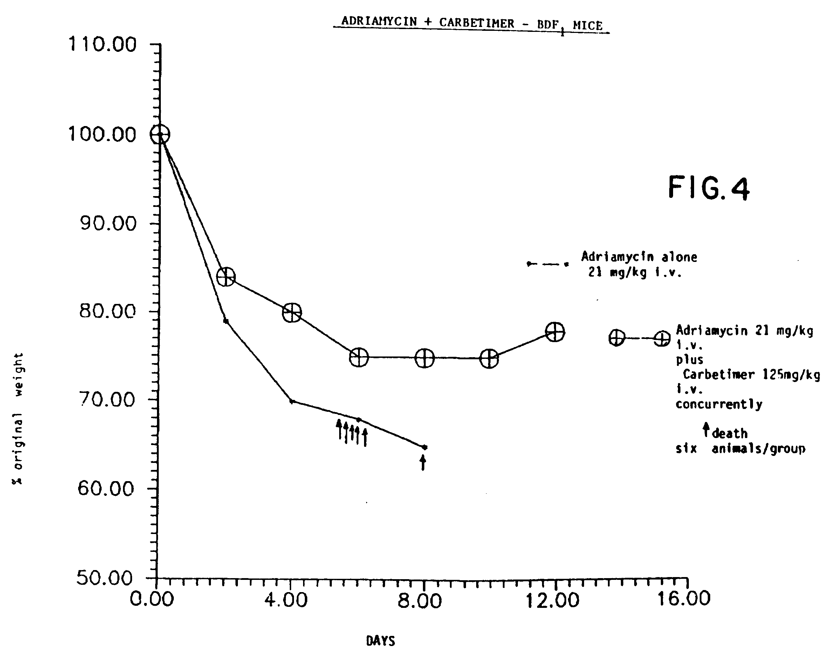

The effect of carbetimer on sub-acute adriamycin toxicity in normal BDF₁ mice was evaluated. Normal BDF₁ mice were treated either with adriamycin alone at 21 mg/kg i.v. or with adriamycin at 21 mg/kg i.v. plus carbetimer 125 mg/kg i.v. given concurrently. The animals treated with a lethal dose of adriamycin died within 10 days of the start of the therapy. In the animal receiving both carbetimer and adriamycin death was prevented. The animals experienced a significant weight loss, but began regaining lost weight by day 14. Data are reported in Table IV in absolute weights of animals and depicted in Figure 4 in terms of percent of original weights of the animals.

**Table IV**

| ADRIAMYCIN + CARBETIMER - BDF₁ MICE | | |
|---|---|---|
| Day | Wt. of animals (gm) | |
| | Adriamycin alone 21 mg/kg i.v. | Adriamycin 21 mg/kg i.v. -plus-Carbetimer at 125 mg i.v. |
| 0 | 23 ± 1 | 23 ± 1 |
| 2 | 18 ± 0.8 | 19 ± 0.8 |
| 4 | 16 ± 0.6 | 18 ± 0.7 |
| 6 | 15 ± 0.4 all dead | 17 ± 0.8 |
| 8 | 14.95 (One animal) | 17 ± 0.6 |
| 10 | - | 17 ± 0.4 |
| 12 | - | 18 ± 0.8 |

### Trial V

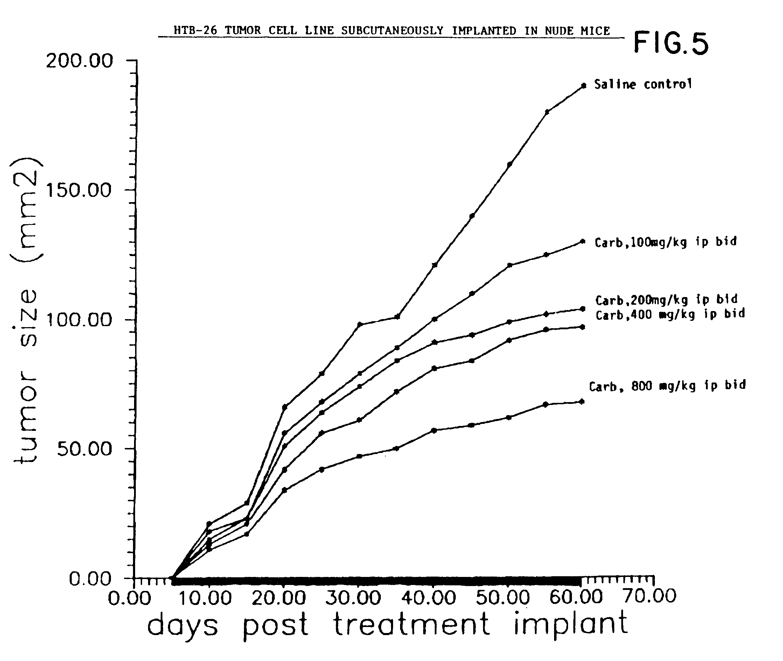

Nude mice were inoculated with HTB 26 cell line. Five days post inoculation of the tumor, treatment was started with saline alone, or carbetimer at 100 mg/kg intraperitoneally b.i.d., or carbetimer 200 mg/kg i.p. b.i.d., or carbetimer 400 mg/kg i.p. b.i.d., or carbetimer 800 mg/kg i.p. b.i.d. The treatment continued for 55 days. Tumor size was measured with the aid of calipers. Dose responses comparing the tumor size with the dose of carbetimer administered are reported in Table V and depicted in Figure 5.

**Table V**

| HTB 26 TUMOR CELL LINE SUB-CUTANEOUSLY IMPLANTED IN NUDE | | | | | |
|---|---|---|---|---|---|
| Days Post-treatment | Saline Alone | Tumor Size (mm²) | | | |
| | | Carbetimer 100 mg/kg i.p. b.i.d. | Carbetimer 200 mg/kg i.p. b.i.d. | Carbetimer 400 mg/kg i.p. b.i.d. | Carbetimer 800 mg/kg i.p. b.i.d. |
| 5 | 0 | 0 | 0 | 0 | 0 |
| 10 | 22 ± 2 | 20 ± 2 | 14 ± 4 | 11 ± 4 | 10 ± 3 |
| 15 | 29 ± 3 | 23 ± 2 | 22 ± 3 | 20 ± 5 | 14 ± 4 |
| 20 | 63 ± 4 | 52 ± 3 | 50 ± 6 | 38 ± 7 | 30 ± 6 |
| 25 | 56 ± 2 | 60 ± 4 | 58 ± 7 | 48 ± 6 | 40 ± 6 |
| 30 | 95 ± 8 | 71 ± 6 | 68 ± 6 | 59 ± 6 | 42 ± 7 |
| 35 | 98 ± 6 | 89 ± 4 | 86 ± 7 | 70 ± 4 | 45 ± 8 |
| 40 | 120 ± 9 | 101 ± 8 | 90 ± 4 | 82 ± 6 | 60 ± 6 |
| 45 | 147 ± 6 | 110 ± 6 | 95 ± 6 | 84 ± 7 | 51 ± 7 |
| 50 | 155 ± 7 | 120 ± 6 | 97 ± 4 | 90 ± 9 | 56 ± 4 |
| 55 | 173 ± 6 | 126 ± 6 | 98 ± 9 | 93 ± 8 | 62 ± 7 |
| 60 | 186 ± 8 | 130 ± 8 | 101 ± 11 | 95 ± 8 | 65 ± 8 |

### Trial VI

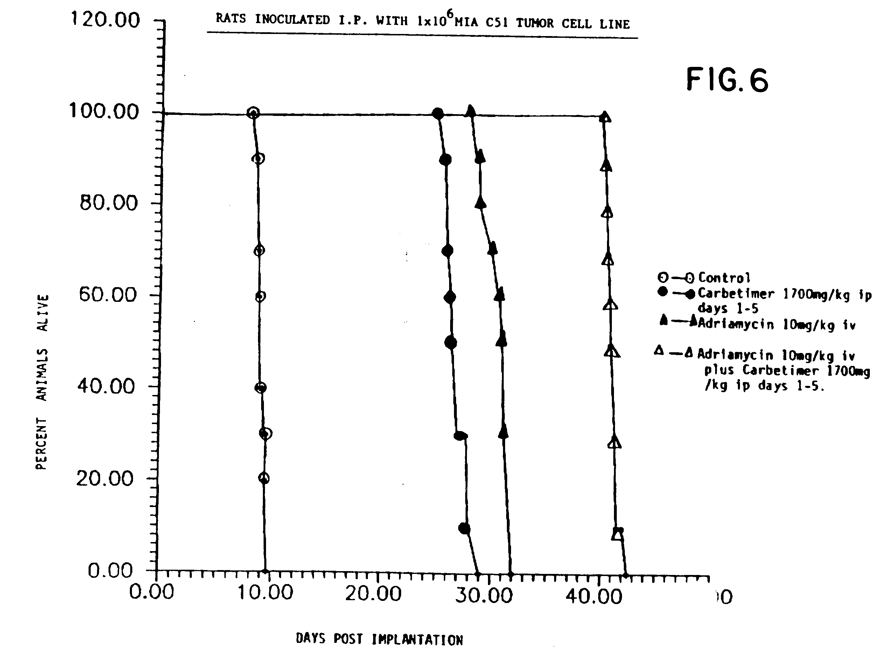

Rats were inoculated with a million cells of MIA C51 cell line. Thereafter, animals were treated with saline control, carbetimer alone at 1700 mg/kg in days 1-5, or adriamycin at 10 mg/kg i.v., or adriamycin 10 mg/kg i.v. plus carbetimer at 1700 mg/kg i.p. Animals treated with the combination of carbetimer and adriamycin remained alive 40 days after treatment. The combination therapy provided greater tumor remission than carbetimer alone or adriamycin alone, indicating two drugs may act in an additive or synergistic manner to cure tumors in tumor-bearing rats. Data are reported in Table VI and depicted in Figure 6.

**Table VI**

| RATS INOCULATED I.P. 1 X 10⁶ MIA C51 TUMOR CELL LINE | | | | |
|---|---|---|---|---|
| Days | Control | % Rats Alive After Treatment | | |
| | | Carbetimer 1700 mg i.p. | Adriamycin 10 mg/kg i.v. Day One Only | Adriamycin 10 mg/kg i.v. Day One Only + Carbetimer 1700 mg/kg i.p. Days 1-5 |
| 0-8 | 100 | 100 | 100 | 100 |
| 9-10 | 0 | 100 | 100 | 100 |
| 11-26 | 0 | 100 | 100 | 100 |
| 27-29 | 0 | 0 | 80 | 100 |
| 30-32 | 0 | 0 | 0 | 100 |
| 33-40 | 0 | 0 | 0 | 100 |
| 41-43 | 0 | 0 | 0 | 0 |

## Claims (Claims for the following Contracting State(s): AT, BE, DE, DK, FR, GB, IT, LU, NL, SE, CH)

1. A combination comprising a cytoprotective copolymer and one or more directly-acting antimetabolite-type antineoplastic agents, wherein said cytoprotective copolymer is a half-amide: half-imide copolymer comprised of monomeric units of
(a) half-amide, half-carboxyl group of the formula 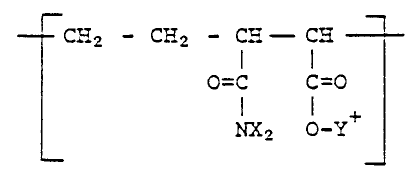 and
(b) imide of the formula 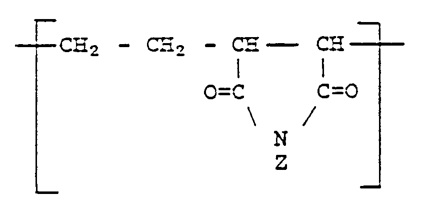
wherein X is independently selected from hydrido and C₁-C₄ alkyl;wherein Y is selected from hydrido, ammonium and pharmaceutically-acceptable metal cations; and wherein Z is selected from hydrido, C₁-C₄ alkyl, amonium and phamaceutically-acceptable metal cations.

2. The combination of Claim 1 wherein said cytoprotective half-amide:half-imide copolymer is a copolymer of at least one olefin monomer having from 2 to 4 carbon atoms and at least one α β-unsaturated polycarboxylic anhydride having from 4 to 6 carbon atoms, having an average molecular weight of from 300 to 1800 and derivatized to obtain both (a) half-amide, half-carboxyl acid groups and (b) imide groups wherein said imide groups comprise from 5% by weight to 40% by weight of said derivatized groups, and the N-alkylated derivatives and pharmaceutically acceptable cationic salt derivatives of said derivatized copolymer, said N-alkylated derivatives having from 1 to 4 carbon atoms in the alkyl substituents.

3. The combination of Claim 2 wherein said cytoprotective half-amide:half-imide copolymer is a copolymer of ethylene and maleic anhydride comprised of
(a) half-amide, half-ammonia salt of the formula 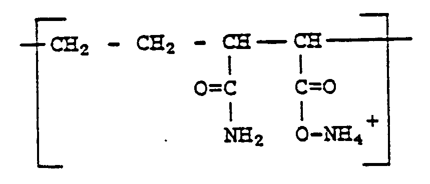 and
(b) unsubstituted imide of the formula

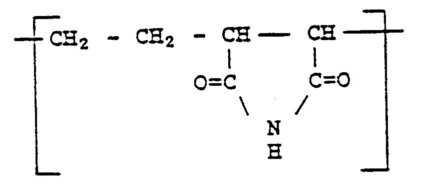

4. The combination of Claim 3 wherein said half-amide:half-imide copolymer is of the formula 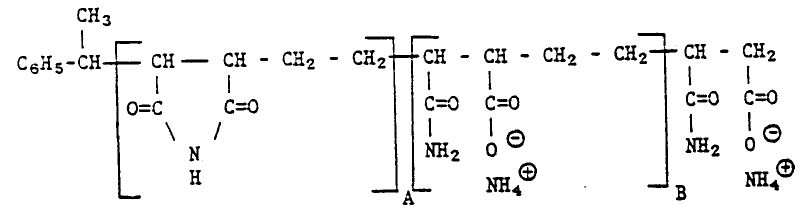 wherein the ratio of A to B is in a range from 1:2 to 1:5.

5. The combination of Claim 4 wherein said half-amide half-imide copolymer is carbetimer.

6. The combination of Claim 1 wherein the antimetabolite antineoplastic agent is selected from the group consisting of 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT and uricytin.

7. The combination of Claim 1 wherein said directly-acting agent is an alkylating-type antineoplastic agent.

8. The combination of Claim 7 wherein said alkylating-type antineoplastic agent is selected from the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsulfam, ifosfamide, iproplatin, lomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromustine, Tanabe Seiyaku TA-077, tauromustine, temozolomide, teroxirone, tetraplatin and trimelamol.

9. The combination of Claim 1 wherein said directly-acting agent is an antibiotic-type antineoplastic agent.

10. The combination of Claim 8 wherein said antibiotic-type antineoplastic agent is selected from the group consisting of Taiho 4181-A, aclarubicin, actinomycin D, actinoplanone, Erbamont ADR-456, aeroplysinin derivative, Ajinomoto AN-201-II, Ajinomoto AN-3, Nippon Soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb, Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindamycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-25024 and zorubicin.

11. The combination of Claim 10 wherein said antibiotic-type antineoplastic agent is an anthraquinone compound.

12. The combination of Claim 11 wherein said anthraquinone compound is an anthracycline compound.

13. The combination of Claim 12 wherein said anthracycline compound is selected from the group consisting of aclarubicin, daunorubicin, ditrisarubicin, doxorubicin, epirubicin, esorubicin, idarubicin, pirarubicin, rodorubicin and zorubicin.

14. The combination of Claim 12 wherein said anthracycline compound is produced by a bacterium of the genus *Streptomycetes*.

15. The combination of Claim 12 wherein said anthracycline is of the formula 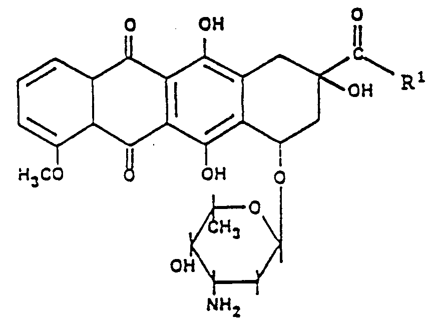 wherein R¹ is selected from C₁-C₅ straight or branched chain alkyl and C₁-C₅ hydroxyalkyl.

16. The combination of Claim 15 wherein R¹ is selected from methyl, ethyl, n-propyl, isopropyl, n-butyl, isobutyl, sec-butyl, tert-butyl, n-pentyl, iso-pentyl, neopentyl, hydroxymethyl, hydroxyethyl, hydroxypropyl, hydroxybutyl and hydroxpentyl.

17. The combination of Claim 16 wherein said anthracycline compound is daunorubicin.

18. The combination or Claim 16 wherein said anthracycline compound is doxorubicin.

19. The combination of Claim 1 wherein the active agent is selected from the group consisting of alpha-carotene, alpha-difluoromethyl-arginine, acitretin, Biotec AD-5, Kyorin AHC-52, alstonine, amonafide, amphethinile, amsacrine, Angiostat, ankinomycin, antineoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristo-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, Curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, elliprabin, elliptinium acetate, Tsumura EPMTC, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyarea, BTG ICRF-187, ilmofosine, isoglutamine, isotretinoin, Otsuka JI-36, Ramot K-477, Otsuak K-76COONa, Kureha Chemical K-AM, MECT Corp. KI-8110, American Cyanamid L-623, leukoregulin, lonidamine, Lundbeck LU-23-112, Lilly LY-186641, NCI (US) MAP, marycin, Merrel Dow MDL-27048, MDL-27048, Medco MEDR-340, merbarone, merocyanine derivatives, methylanilinoacridine, Molecular Genetics MGI-136, minactivin, mitonafide, mitoquidone, mitotane, mopidamol, motretinide, Zenyaku Kogyo MST-16, N-(retinoyl)amino acids, Nisshin Flour Milling N-021, N-acylated-dehydroalanines, nafasatrom, Taisho NCU-190, nocodazole derivative, Normosang, NCI NSC-145813, NCI NSC-361456, NCI NSC-604782, NCI NSC-95580, octreotide, Ono ONO-112, oquizanocine, Akzo Org-10172, pancratistatin, pazelliptine, Warner-Lambert PD-111707, Warner-Lambert PD-115934, Warner-Lambert PD-131141, Pierre Fabre PE-1001, ICRT peptide D, piroxantrone, polyhaematoporphyrin, polypreic acid, Efamol porphyrin, probimane, procarbazine, proglumide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, Topostin, Teijin TT-82, Kyowa Hakko UCN-01, Kyowa Hakko UCN-1028, ukrain, Eastman Kodak USB-006, vinblastine sulfate, vincristine, vindesine, vinestramide and vinorelbine, vintriptol, vinzolidine, withanolides, Yamanouchi YM-534.

20. A combination according to Claim 1 wherein said cyto-protective copolymer is carbetimer and wherein the directly acting agent is an anthracycline-type antineoplastic agent.

21. The combination of Claim 20 wherein said anthracycline-type antineoplastic agent is selected from the group consisting of aclarubicin, daunorubicin, ditrisarubicin, doxorubicin, epirubicin, esorubicin, idarubicin, pirarubicin, rodorubicin and zorubicin.

22. The combination of Claim 21 wherein said anthracycline-type antineoplastic agent is doxorubicin.

23. Use of an antineoplastic agent and of carbetimer for preparing a medicament for treating neoplasia in a patient.

24. Use according to Claim 23 for preparing a medicament for treating acute leukemias, malignant lymphomas, Hodgkin's disease, ovarian carcinoma, breast carcinoma, lung small cell carcinoma, osteogenic carcinoma, Ewing carcinoma, soft-tissue carcinoma, metastatic breast aderocarcinoma, bladder carcinoma, bronchogenic carcinoma, neuroblastoma, metastatic thyroid carcinoma, endometrium carcinoma, testes carcinoma, prostate carcinoma, cervix carcinoma, head and neck carcinoma, gastric carcinoma and plasma-cell myeloma.

25. Use according to Claim 24 wherein said antineoplastic agent is an anthracycline-type antineoplastic agent.

26. Use according to Claim 25 wherein said anthracycline-type antineoplastic agent is selected from the group consisting of aclarubicin, daunorubicin, ditrisarubicin, doxorubicin, epirubicin, esorubicin, idarubicin, pirarubicin, rodorubicin and zorubicin.

27. Use according to Claim 26 wherein said anthracycline-type antineoplastic agent is doxorubicin.

## Claims (Claims for the following Contracting State(s): ES, GR)

1. Use of a cytoprotective copolymer and one or more directly-acting antimetabolite-type antineoplastic agents for preparing a medicament for treating neoplasia in a patient, wherein said cyto-protective copolymer is a half-amide:half-imide copolymer comprised of monomeric units of
(a) half-amide, half-carboxyl group of the formula 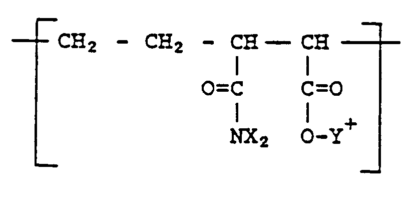 and
(b) imide of the formula 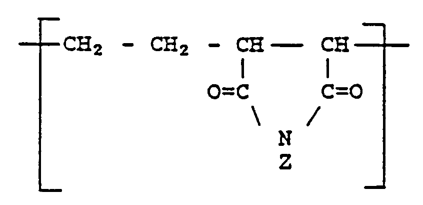
wherein X is independently selected from hydrido and C₁-C₄ alkyl; wherein Y is selected from hydrido, ammonium and pharmaceutically-acceptable metal cations; and wherein Z is selected from hydrido, C₁-C₄ alkyl, ammonium and phamaceutically-acceptable metal cations.

2. Use according to Claim 1 wherein said cytoprotective half-amide:half-imide copolymer is a copolymer of at least one olefin monomer having from 2 to 4 carbon atoms and at least one α β-unsaturated polycarboxylic anhydride having from 4 to 6 carbon atoms, having an average molecular weight of from 300 to 1800 and derivatized to obtain both (a) half-amide, half-carboxyl acid groups and (b) imide groups wherein said imide groups comprise from 5% by weight to 40% by weight of said derivatized groups, and the N-alkylated derivatives and pharmaceutically acceptable cationic salt derivatives of said derivatized copolymer, said N-alkylated derivatives having from 1 to 4 carbon atoms in the alkyl substituents.

3. Use according to Claim 2 wherein said cytoprotective half-amide:half-imide copolymer is a copolymer of ethylene and maleic anhydride comprised of
(a) half-amide, half-ammonium salt of the formula 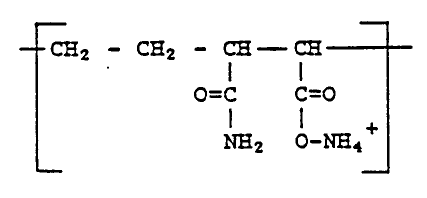 and
(b) unsubstituted imide of the formula

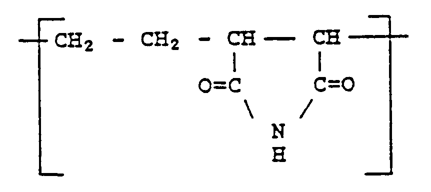

4. Use according to Claim 3 wherein said half-amide:half-imide copolymer is of the formula 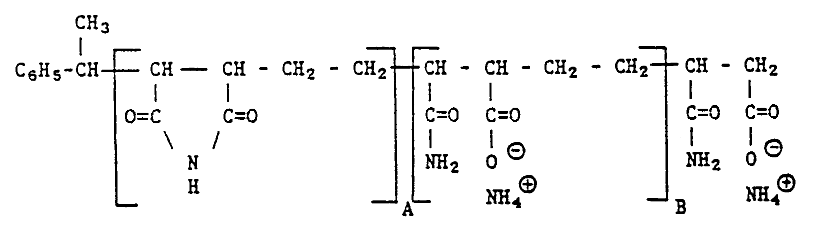 wherein the ratio of A to B is in a range from 1:2 to 1:5.

5. Use according to Claim 4 wherein said half-amide:half imide copolymer is carbetimer.

6. Use according to Claim 1 wherein the antimetabolite antineoplastic agent is selected from the group consisting of 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT and uricytin.

7. Use according to Claim 1 wherein said directly-acting agent is an alkylating-type antineoplastic agent.

8. Use according to Claim 7 wherein said alkylating-type antineoplastic agent is selected from the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsulfam, ifosfamide, iproplatin, lomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromustine, Tanabe Seiyaku TA-077, tauromustine, temozolomide, teroxirone, tetraplatin and trimelamol.

9. Use according to Claim 1 wherein said directly acting agent is an antibiotic-type antineoplastic agent.

10. Use according to Claim 9 wherein said antibiotic-type antineoplastic agent is selected from the group consisting of Taiho 4181-A, aclarubicin, actinomycin D, actinoplanone, Erbamont ADR-456, aeroplysinin derivative, Ajinomoto AN-201-II, Ajinomoto AN-3, Nippon Soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb, Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindamycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-25024 and zorubicin.

11. Use according to Claim 10 wherein said antibiotic-type antineoplastic agent is an anthraquinone compound.

12. Use according to Claim 11 wherein said anthraquinone compound is an anthracycline compound.

13. Use according to Claim 12 wherein said anthracycline compound is selected from the group consisting of aclarubicin, daunorubicin, ditrisarubicin, doxorubicin, epirubicin, esorubicin, idarubicin, pirarubicin, rodorubicin and zorubicin.

14. Use according to Claim 12 wherein said anthracycline compound is produced by a bacterium of the genus *Streptomycetes*.

15. Use according to Claim 12 wherein said anthracycline is of the formula 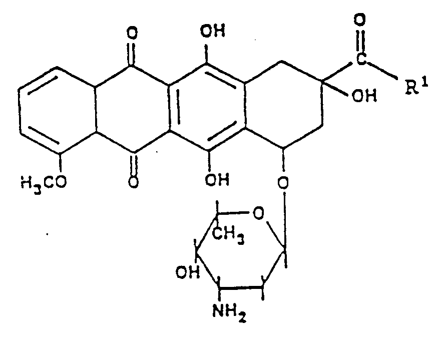 wherein R¹ is selected from straight or branched chain C₁-C₅ alkyl and C₁-C₅ hydroxyalkyl.

16. Use according to Claim 15 wherein R¹ is selected from methyl, ethyl, n-propyl, isopropyl, n-butyl, isobutyl, sec-butyl, tert-butyl, n-pentyl, iso-pentyl, neopentyl, hydroxymethyl, hydroxyethyl, hydroxypropyl, hydroxybutyl and hydroxypentyl.

17. Use according to Claim 16 wherein said anthracycline compound is daunorubicin.

18. Use according to Claim 17 wherein said anthracycline compound is doxorubicin.

19. Use according to Claim 1 wherein said directly-acting agent is selected from the group consisting of alpha-carotene, alpha-difluoromethyl-arginine, acitretin, Biotec AD-5, Kyorin AHC-52, alstonine, amonafide, amphethinile, amsacrine, Angiostat, ankinomycin, antineoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristo-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, Curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DH-341, Toyo Pharmar DM-75, Daiichi Seiyaku DN-9693, elliprabin, elliptinium acetate, Tsumura EPMTC, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyarea, BTG ICRF-187, ilmofosine, isoglutamine, isotretinoin, Otsuka JI-36, Ramot K-477, Otsuak K-76COONa, Kureha Chemical K-AM, MECT Corp. KI-8110, American Cyanamid L-623, leukoregulin, lonidamine, Lundbeck LU-23-112, Lilly LY-186641, NCI (US) MAP, marycin, Merrel Dow MDL-27048, MDL-27048, Medco MEDR-340, merbarone, merocyanine derivatives, methylanilinoacridine, Molecular Genetics MGI-136, minactivin, mitonafide, mitoquidone, mitotane, mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, octreotide, Ono ONO-112, oquizanocine, Akzo Org-10172, pancratistatin, pazelliptine, Warner-Lambert PD-111707, Warner-Lambert PD-115934, Warner-Lambert PD-131141, Pierre Fabre PE-1001, ICRT peptide D, piroxantrone, polyhaematoporphyrin, polypreic acid, Efamol porohyrin, probimane, procarbazine, proglumide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogeranium, Unimed, SS Pharmaceutical SS-554, strypoldinone, Stypolcione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, Topostin, Teijin TT-82, Kyowa Hakko UCN-01, Kyowa Hakko UCN-1028, ukrain, Eastman Kodak USB-006, vinblastine sulfate, vincristine, vindesine, vinestramide and vinorelbine, vintriptol, vinzolidine, withanolides, Yamanouchi YM-534.

20. Use according to Claim 1 wherein said cyctsprotective copolymer is carbetimer and the directly acting agent is an anthracycline-type antineoplastic agent.

21. Use according to Claim 20 wherein said anthracycline-type antineoplastic agent is selected from the group consisting of aclarubicin, daunorubicin, ditrisarubicin, doxorubicin, epirubicin, esorubicin, icarubicin, pirarubicin, rodorubicin and zorubicin.

22. Use according to Claim 21 wherein said anthracycline-type antineoplastic agent is doxorubicin.

23. Use according to Claim 1 wherein said cytoprotective copolymer is carbetimer.

24. Use according to Claim 23 for preparing a medicament for treating acute leukemias, malginant lymphomas, Hodgkin's disease, ovarian carcinoma, breast carcinoma, lung small cell carcinoma, osteogenic carcinoma, Ewing carcinoma, soft-tissue carcinoma, metastatic breast aderocarcinoma, bladder carcinoma, bronchogenic carcinoma, neuroblastama, metastatic thyroid carcinoma, endometrium carcinoma, testes carcinoma, prostate carcinoma, cervix carcinoma, head and neck carcinoma, gastric carcinoma and plasma-cell myeloma.

25. Use according to Claim 24 wherein said directly acting agent is an anthracycline-type antineoplastic agent.

26. Use according to Claim 25 wherein said anthracycline-type antineoplastic agent is selected from the group consisting of aclarubicin, daunorubicin, ditrisarubicin, doxorubicin, epirubicin, esorubicin, idarubicin, pirarubicin, rodorubicin and zorubicin.

## Patentansprüche (Patentansprüche für folgende(n) Vertragsstaat(en): AT, BE, DE DK, FR, GB, IT, LU, NL, SE, CH)

1. Kombination, umfassend ein cytoprotektives Copolymer und ein oder mehrere direkt wirkende Antineoplastika vom Typ Antimetabolit, wobei das cytoprotektive Copolymer ein Halb-Amid:Halb-Imid-Copolymer ist, bestehend aus monomeren Einheiten von (a) Halb-Amid/Halb-Carboxyl-gruppe der Formel 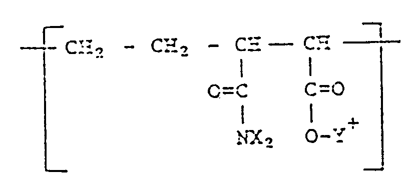 und (b) Imid der Formel 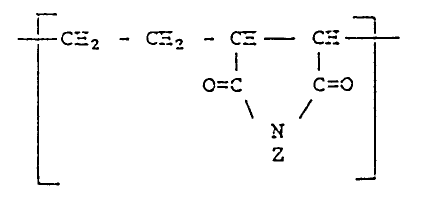 worin X unabhängig aus Hydrido und C₁-C₄-Alkyl ausgewählt ist; worin Y aus Hydrido, Ammonium und pharmazeutisch unbedenklichen Metallkationen ausgewählt ist; und worin Z aus Hydrido, C₁-C₄-Alkyl, Ammonium und pharmazeutisch unbedenklichen Metallkationen ausgewählt ist.

2. Kombination nach Anspruch 1, worin das cytoprotektive Halb-Amid:Halb-Imid-Copolymer ein Copolymer aus zumindest einem Olefinmonomer mit 2 bis 4 Kohlenstoffatomen und zumindest einem α,β-ungesättigten Polycarbonsäureanhydrid mit 4 bis 6 Kohlenstoffatomen, mit einem durchschnittlichen Molekulargewicht von 300 bis 1800 und derivatisiert ist, um (a) Halb-Amid/Halb-Carbonsäure-Gruppen und (b) Imidgruppen zu erhalten, wobei die Imidgruppen 5 Gew.-% bis 40 Gew.-% der derivatisierten Gruppen umfassen, und die N-alkylierten Derivate und pharmazeutisch unbedenklichen kationischen Salzderivate des derivatisierten Copolymers, wobei die N-alkylierten Derivate 1 bis 4 Kohlenstoffatome in den Alkylsubstituenten aufweisen.

3. Kombination nach Anspruch 2, worin das cytoprotektive Halb-Amid:Halb-Imid-Copolymer ein Copolymer von Ethylen und Maleinsäureanhydrid ist, bestehend aus (a) Halb-Amid/Halb-Ammonium-salz der Formel 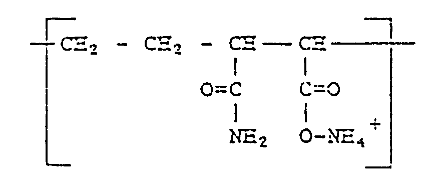 und (b) unsubstituiertem Imid der Formel

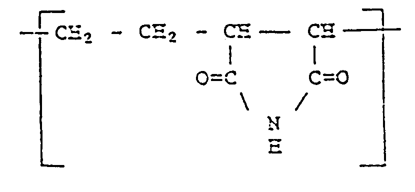

4. Kombination nach Anspruch 3, worin das Halb-Amid:Halb-Imid-Copolymer die Formel 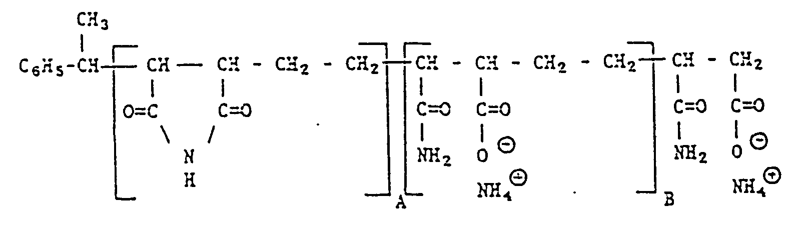 aufweist, worin das Verhältnis A zu B im Bereich von 1:2 bis 1:5 liegt.

5. Kombination nach Anspruch 4, worin das Halb-Amid:Halb-Imid-Copolymer Carbetimer ist.

6. Kombination nach Anspruch 1, worin das Antineoplastikum vom Typ Antimetabolit aus der aus 5-FU-Fibrinogen, Acanthifolsäure, Aminothiadiazol, Brequinar-Natrium, Carmofur, Ciba-Geigy CGP-30694, Cyclopentylcytosin, Cytarabinphosphatstearat, Cytarabin-Konjugaten, Lilly DATHF, Merrel Dow DDFC, Dezaguanin, Didesoxycytidin, Didesoxyguanosin, Didox, Yoshitomi DMDC, Doxifluridin, Wellcome EHNA, Merck & Co. EX-015, Fazarabin, Floxuridin, Fludarabinphosphat, 5-Fluoro-uracil, N-(2'-Furanidyl)-5-fluoro-uracil, Daiichi Seiyaku FO-152, Isopropylpyrrolizin, Lilly LY-188011, Lilly LY-264618, Methobenzaprim, Methotrexat, Wellcome MZPES, Norspermidin, NCI NSC-127716, NCI NSC-264880, NCI NSC-39661, NCI NSC-612567, Warner-Lambert PALA, Pentostatin, Piritrexim, Plicamycin, Asahi Chemical PL-AC, Takeda TAC-788, Thioguanin, Tiazofurin, Erbamont TIF, Trimetrexat, Tyrosin-Kinase-Inhibitoren, Tyrosin-Proteinkinase-Inhibitoren, Taiho UFT und Uricytin bestehenden Gruppe ausgewählt ist.

7. Kombination nach Anspruch 1, worin das direct wirkende Mittel ein Antineoplastikum vom Typ Alkylierungsmittel ist.

8. Kombination nach Anspruch 7, worin das Antineoplastikum vom Typ Alkylierungsmittel aus der aus Shionogi 254-S, Aldo-Phosphamid-Analogen, Altretamin, Anaxiron, Boehringer Mannheim BBR-2207, Bestrabucil, Budotitan, Wakunaga CA-102, Carboplatin, Carmustin, Chinoin-139, Chinoin-153, Chlorambucil, Cisplatin, Cyclophosphamid, American Cyanamid CL-286558, Sanofi CY-233, Cyplatat, Degussa D-19-384, Sumitomo DACHP(Myr)2, Diphenylspiromustin, Diplatinum-Cytostatikum, Erba Distamycin-Derivaten, Chugai DWA-2114R, ITI E09, Elmustin, Erbamont FCE-24517, Estramustinphosphat-Natrium, Fotemustin, Unimed G-6-M, Chinoin GYKI-17230, Hepsulfam, Ifosfamid, Iproplatin, Lomustin, Mafosfamid, Mitolactol, Nippon Kayaku NK-121, NCI NSC-264395, NCI NSC-342215, Oxaliplatin, Upjohn PCNU, Prednimustin, Proter PTT-119, Ranimustin, Semustin, SmithKline SK&F-101772, Yakult Honsha SN-22, Spiromustin, Tanabe Seiyaku TA-077, Tauromustin, Temozolomid, Teroxiron, Tetraplatin und Trimelamol bestehenden Gruppe ausgewählt ist.

9. Kombination nach Anspruch 1, worin das direkt wirkende Mittel ein Antineoplastikum vom Typ Antibiotikum ist.

10. Kombination nach Anspruch 8, worin das Antineoplastikum vom Typ Antibiotikum aus der aus Taiho 4181-A, Aclarubicin, Actinomycin D, Actinoplanon, Erbamont ADR-456, Aeroplysinin-Derivat, Ajinomoto AN-201-II, Ajinomoto AN-3, Nippon Soda Anisomycinen, Anthracyclin, Azino-Mycin-A, Bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, Bleomycinsulfat, Bryostatin-1, Taiho C-1027, Calichemycin, Chromoximycin, Dactinomycin, Daunorubicin, Kyowa Hakko DC-102, Kyowa Hakko DC-79, Kyowa Hakko DC-88A, Kyowa Hakko DC89-A1, Kyowa Hakko DC92-B, Ditrisarubicin B, Shionogi DOB-41, Doxorubicin, Doxorubicin-Fibrinogen, Elsamicin A, Epirubicin, Erbstatin, Esorubicin, Esperamicin-A1, Esperamicin-Alb, Erbamont FCE-21954, Fujisawa FK-973, Fostriecin, Fujisawa FR-900482, Glidobactin, Gregatin-A, Grincamycin, Herbimycin, Idarubicin, Illudinen, Kazusamycin, Kesarirhodinen, Kyowa Hakko KM-5539, Kirin Brewery KRN-8602,KyowaHakkoKT-5432,KyowaHakkoKT-5594,KyowaHakkoKT-6149,AmericanCyanamid LL-D49194, Meiji Seika ME 2303, Menogaril, Mitomycin, Mitoxantron, SmithKline M-TAG, Neoenactin, Nippon Kayaku NK-313, Nippon Kayaku NKT-01, SRI International NSC-357704, Oxalysin, Oxaunomycin, Peplomycin, Pilatin, Pirarubicin, Porothramycin, Pyrindamycin A, Tobishi PA-I, Rapamycin, Rhizoxin, Rodorubicin, Sibanomicin, Siwenmycin, Sumitomo SM-5887, Snow Brand SN-706,SnowBrandSN-07,Sorangicin-A,Sparsomycin,SSPharmaceuticalSS-21020,SSPharmaceutical SS-7313B, SS Pharmaceutical SS-9816B, Steffimycin B, Taiho 4181-2, Talisomycin, Takeda TAN-868A, Terpentecin, Thrazin, Tricrozarin A, Upjohn U-73975, Kyowa Hakko UCN-10028A, Fujisawa WF-3405, Yoshitomi Y-25024 und Zorubicin bestehenden Gruppe ausgewählt ist.

11. Kombination nach Anspruch 10, worin das Antineoplastikum vom Typ Antibiotikum eine Anthrachinon-Verbindung ist.

12. Kombination nach Anspruch 11, worin die Anthrachinon-Verbindung eine Anthracyclin-Verbindung ist.

13. Kombination nach Anspruch 12, worin die Anthracyclin-Verbindung aus der aus Aclarubicin, Daunorubicin, Ditrisarubicin, Doxorubicin, Epirubicin, Esorubicin, Idarubicin, Pirarubicin, Rodorubicin und Zorubicin bestehenden Gruppe ausgewählt ist.

14. Kombination nach Anspruch 12, worin die Anthracyclin-Verbindung durch ein Bakterium der Gattung Streptomycetes produziert wird.

15. Kombination nach Anspruch 12, worin das Anthracyclin die Formel 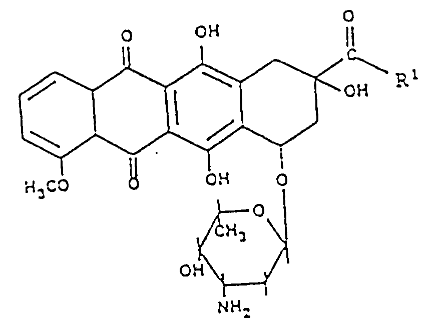 hat, worin R¹ aus geradkettigem oder verzweigtem C₁-C₅-Alkyl und C₁-C₅-Hydroxyalkyl ausgewählt ist.

16. Kombination nach Anspruch 15, worin R¹ aus Methyl, Ethyl, n-Propyl, Isopropyl, n-Butyl, Isobutyl, sec-Butyl, tert-Butyl, n-Pentyl, Isopentyl, Neopentyl, Hydroxymethyl, Hydroxyethyl, Hydroxypropyl, Hydroxybutyl und Hydroxypentyl ausgewählt ist.

17. Kombination nach Anspruch 16, worin die Anthracyclin-Verbindung Daunorubicin ist.

18. Kombination nach Anspruch 16, worin die Anthracyclin-Verbindung Doxorubicin ist.

19. Kombination nach Anspruch 1, worin das aktive Mittel aus der aus alpha-Caroten, alpha-Difluormethylarginin, Acitretin, Biotec AD-5, Kyorin AHC-52, Alstonin, Amonafid, Amphethinil, Amsacrin, Angiostat, Ankinomycin, Antineoplaston A10, Antineoplaston A2, Antineoplaston A3, Antineoplaston A5, Antineoplaston AS2-1, Henkel APD, Aphidicolinglycinat, Asparaginase, Avarol, Baccharin, Batracyclin, Benfluron, Benzotript, Ipsen-Beaufour BIM-23015, Bisantren, Bristol-Myers BMY-40481, Vestar Boron-10, Bromofosfamid, Wellcome BW-502, Wellcome BW-773, Caracemid, Carmethizol-hydrochlorid, Ajinomoto CDAF, Chlorsulfachinoxalon, Chemes CHX-2053, Chemex CHX-100, Warner-Lambert CI-921, Warner-Lambert CI-937, Warner-Lambert CI-941, Warner-Lambert CI-958, Clanfenur, Claviridenon, ICN Verbindung 1259, ICN Verbindung 4711, Contracan, Yakult Honsha CPT-11, Crisnatol, Curaderm, Cytochalasin B, Cytarabin, Cytocytin, Merz D-609, DABIS Maleat, Dacarbazin, Datelliptinium, Didemnin-B, Dihämatoporphyrin-ether, Dihydrolenperon, Dinalin, Distamycin, Toyo Pharmar DM-341, Toyo Pharmar DM-75, Daiichi Seiyaku DN-9693, Elliprabin, Elliptiniumacetat, Tsumura EPMTC, Ergotamin, Etoposid, Etretinat, Fenretinid, Fujisawa FR-57704, Galliumnitrat, Genkwadaphnin, Chugai GLA-43, Glaxo GR-63178, Grifolan NMF-5N, Hexadecylphosphocholin, Green Cross HO-221, Homoharringtonin, Hydroxyharnstoff, BTG ICRF-187, Ilmofosin, Isoglutamin, Isotretinoin, Otsuka JI-36, Ramot K-477,OtsuakK-76COONa,KurehaChemicalK-AM,MECTCorp.KI-8110,AmericanCyanamidL-623, Leukoregulin, Lonidamin, Lundbeck LU-23-112, Lilly LY-186641, NCI (US) MAP, Marycin, Merrel Dow MDL-27048, MDL-27048, Medco MEDR-340, Merbaron, Merocyanin-Derivaten, Methylanilinoacridin, Molecular Genetics MGI-136, Minactivin, Mitonafid, Mitoquidon, Mitotan, Mopidamol, Motretinid, Zenyaku Kogyo MST-16, N-(Retinoyl)aminosäuren, Nisshin Flour Milling N-021, N-acelierten Dehydroalaninen, Nafazatrom, Taisho NCU-190, Nocodazol-Derivat, Normosang, NCI NSC-145813, NCI NSC-361456, NCI NSC-604782, NCI NSC-95580, Octreotid, Ono ONO-112, Oquizanocin, Akzo Org-10172, Pancratistatin, Pazelliptin, Warner-Lambert PD-111707, Warner-Lambert PD-115934, Warner-Lambert PD-131141, Pierre Fabre PE-1001, ICRT Peptid D, Piroxantron, Polyhämatoporphyrin, polypreic-Säure Efamol-Porphyrin, Probiman, Procarbazin, Proglumid, Invitron Protease-Nexin I, Tobishi RA-700, Razoxan, Sapporo Breweries RBS, Restrictin-P, Retelliptin, Retinsäure, Rhone-Poulenc RP-49532, Rhone-Poulenc RP-56976, SmithKline SK&F-104864, Sumitomo SM-108, Kuraray SMANCS, SeaPharm SP-10094, Spatol, Spirocyclopropan-Derivaten, Spirogermanium, Unimed, SS Pharmaceutical SS-554, Strypoldinon, Stypoldion, Suntory SUN 0237, Suntory SUN 2071, Superoxid-Dismutase, Toyama T-506, Toyama T-680, Taxol, Teijin TEI-0303, Teniposid, Thaliblastin, Eastman Kodak TJB-29, Tocotrienol, Topostin, Teijin TT-82, Kyowa Hakko UCN-01, Kyowa Hakko UCN-1028, Ukrain, Eastman Kodak USB-006, Vinblastinsulfat, Vincristin, Vindesin, Vinestramid und Vinorelbin, Vintriptol, Vinzolidin, Withanoliden, Yamanouchi YM-534 bestehenden Gruppe ausgewählt ist.

20. Kombination nach Anspruch 1, worin das cytoprotektive Copolymer Carbetimer ist und das direkt wirkende Mittel ein Antineoplastikum vom Typ Anthracyclin ist.

21. Kombination nach Anspruch 20, worin das Antineoplastikum vom Typ Anthracyclin aus der aus Aclarubicin, Daunorubicin, Ditrisarubicin, Doxorubicin, Epirubicin, Esorubicin, Idarubicin, Pirarubicin, Rodorubicin und Zorubicin bestehenden Gruppe ausgewählt ist.

22. Kombination nach Anspruch 21, worin das Antineoplastikum vom Typ Anthracyclin Doxorubicin ist.

23. Verwendung eines Antineoplastikums und von Carbetimer zur Herstellung eines Arzneimittels zur Behandlung von Neoplasie bei einem Patienten.

24. Verwendung nach Anspruch 23 zur Herstellung eines Arzneimittels zur Behandlung akuter Leukämien, maligner Lymphome, der Hodgkin-Krankheit, von Ovarialkarzinom, Brustkrebs, Kleinzellenkarzinom der Lunge, osteogenem Karzinom, Ewing-Karzinom, Weichteilkarzinomen, metastatischem Brustadenokarzinom, Blasenkrebs, bronchogenem Karzinom, Neuroblastom, metastatischem Schilddrüsenkarzinom, Endometriumkarzinom, Hodenkrebs, Prostatakarzinom, Zervixkarzinom, Karzinomen im Kopf- und Halsbereich, Magenkarzinom und Plasmazellenmyelom.

25. Verwendung nach Anspruch 24, worin das Antineoplastikum ein Antineoplastikum vom Typ Anthracyclin ist.

26. Verwendung nach Anspruch 25, worin das Antineoplastikum vom Typ Anthracyclin aus der aus Aclarubicin, Daunorubicin, Ditrisarubicin, Doxorubicin, Epirubicin, Esorubicin, Idarubicin, Pirarubicin, Rodorubicin und Zorubicin bestehenden Gruppe ausgewählt ist.

27. Verwendung nach Anspruch 26, worin das Antineoplastikum vom Typ Anthracyclin Doxorubicin ist.

## Patentansprüche (Patentansprüche für folgende(n) Vertragsstaat(en): ES, GR)

1. Verwendung eines cytoprotektiven Copolymers und eines oder mehrerer direkt wirkender Antineoplastika vom Typ Antimetabolit zur Herstellung eines Arzneimittels zur Behandlung von Neoplasie bei einem Patienten, wobei das cytoprotektive Copolymer ein Halb-Amid:Halb-Imid-Copolymer ist, bestehend aus monomeren Einheiten von (a) Halb-Amid/Halb-Carboxylgruppe der Formel 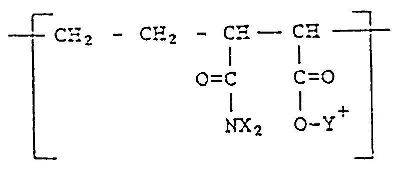 und (b) Imid der Formel 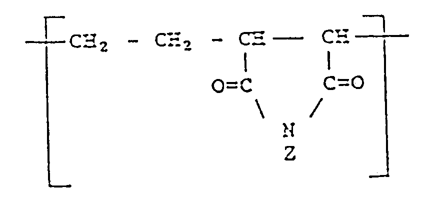 worin X unabhängig aus Hydrido und C₁-C₄-Alkyl ausgewählt ist; worin Y aus Hydrido, Ammonium und pharmazeutisch unbedenklichen Metallkationen ausgewählt ist; und worin Z aus Hydrido, C₁-C₄-Alkyl, Ammonium und pharmazeutisch unbedenklichen Metallkationen ausgewählt ist.

2. Verwendung nach Anspruch 1, worin das cytoprotektive Halb-Amid:Halb-Imid-Copolymer ein Copolymer aus zumindest einem Olefinmonomer mit 2 bis 4 Kohlenstoffatomen und zumindest einem α,β-ungesättigten Polycarbonsäureanhydrid mit 4 bis 6 Kohlenstoffatomen, mit einem durchschnittlichen Molekulargewicht von 300 bis 1800 und derivatisiert ist, um (a) Halb-Amid/Halb-Carbonsäure-Gruppen und (b) Imidgruppen zu erhalten, wobei die Imidgruppen 5 Gew.-% bis 40 Gew.-% der derivatisierten Gruppen umfassen, und die N-alkylierten Derivate und pharmazeutisch unbedenklichen kationischen Salzderivate des derivatisierten Copolymers, wobei die N-alkylierten Derivate 1 bis 4 Kohlenstoffatome in den Alkylsubstituenten aufweisen.

3. Verwendung nach Anspruch 2, worin das cytoprotektive Halb-Amid:Halb-Imid-Copolymer ein Copolymer von Ethylen und Maleinsäureanhydrid ist, bestehend aus (a) Halb-Amid/Halb-Ammoniumsalz der Formel 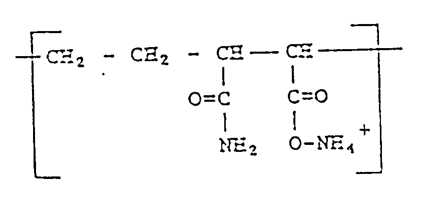 und (b) unsubstituiertem Imid der Formel

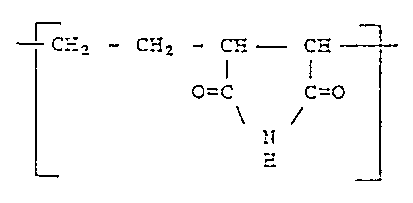

4. Verwendung nach Anspruch 3, worin das Halb-Amid:Halb-Imid-Copolymer die Formel 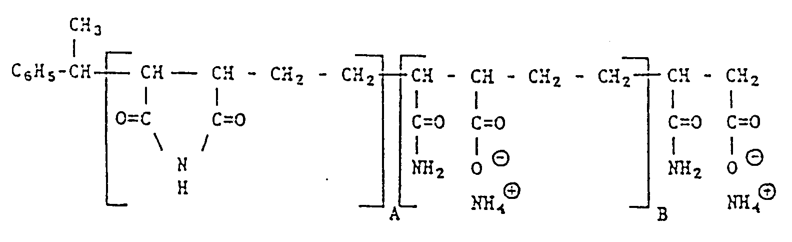 aufweist, worin das Verhältnis A zu B im Bereich von 1:2 bis 1:5 liegt.

5. Verwendung nach Anspruch 4, worin das Halb-Amid:Halb-Imid-Copolymer Carbetimer ist.

6. Verwendung nach Anspruch 1, worin das Antineoplastikum vom Typ Antimetabolit aus der aus 5-FU-Fibrinogen, Acanthifolsäure, Aminothiadiazol, Brequinar-Natrium, Carmofur, Ciba-Geigy CGP-30694, Cyclopentylcytosin, Cytarabinphosphatstearat, Cytarabin-Konjugaten, Lilly DATHF, Merrel Dow DDFC, Dezaguanin, Didesoxycytidin, Didesoxyguanosin, Didox, Yoshitomi DMDC, Doxifluridin, Wellcome EHNA, Merck & Co. EX-015, Fazarabin, Floxuridin, Fludarabinphosphat, 5-Fluoro-uracil, N-(2'-Furanidyl)-5-fluoro-uracil, Daiichi Seiyaku FO-152, Isopropylpyrrolizin, Lilly LY-188011, Lilly LY-264618, Methobenzaprim, Methotrexat, Wellcome MZPES, Norspermidin, NCI NSC-127716, NCI NSC-264880, NCI NSC-39661, NCI NSC-612567, Warner-Lambert PALA, Pentostatin, Piritrexim, Plicamycin, Asahi Chemical PL-AC, Takeda TAC-788, Thioguanin, Tiazofurin, Erbamont TIF, Trimetrexat, Tyrosin-Kinase-Inhibitoren, Tyrosin-Proteinkinase-Inhibitoren, Taiho UFT und Uricytin bestehenden Gruppe ausgewählt ist.

7. Verwendung nach Anspruch 1, worin das direkt wirkende Mittel ein Antineoplastikum vom Typ Alkylierungsmittel ist.

8. Verwendung nach Anspruch 7, worin das Antineoplastikum vom Typ Alkylierungsmittel aus der aus Shionogi 254-S, Aldo-Phosphamid-Analogen, Altretamin, Anaxiron, Boehringer Mannheim BBR-2207, Bestrabucil, Budotitan, Wakunaga CA-102, Carboplatin, Carmustin, Chinoin-139, Chinoin-153, Chlorambucil, Cisplatin, Cyclophosphamid, American Cyanamid CL-286558, Sanofi CY-233, Cyplatat, Degussa D-19-384, Sumimoto DACHP(Myr)2, Diphenylspiromustin, Diplatinum-Cytostatikum, Erba Distamycin-Derivaten, Chugai DWA-2114R, ITI E09, Elmustin, Erbamont FCE-24517, Estramustinphosphat-Natrium, Fotemustin, Unimed G-6-M, Chinoin GYKI-17230, Hepsulfam, Ifosfamid, Iproplatin, Lomustin, Mafosfamid, Mitolactol, Nippon Kayaku NK-121, NCI NSC-264395, NCI NSC-342215, Oxaliplatin, Upjohn PCNU, Prednimustin, Proter PTT-119, Ranimustin, Semustin, SmithKline SK&F-101772, Yakult Honsha SN-22, Spiromustin, Tanabe Seiyaku TA-077, Tauromustin, Temozolomid, Teroxiron, Tetraplatin und Trimelamol bestehenden Gruppe ausgewählt ist.

9. Verwendung nach Anspruch 1, worin das direkt wirkende Mittel ein Antineoplastikum vom Typ Antibiotikum ist.

10. Verwendung nach Anspruch 9, worin das Antineoplastikum vom Typ Antibiotikum aus der aus Taiho 4181-A, Aclarubicin, Actinomycin D, Actinoplanon, Erbamont ADR-456, Aeroplysinin-Derivat, Ajinomoto AN-201-II, Ajinomoto AN-3, Nippon Soda Anisomycinen, Anthracyclin, Azino-Mycin-A, Bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, Bleomycinsulfat, Bryostatin-1, Taiho C-1027, Calichemycin, Chromoximycin, Dactinomycin, Daunorubicin, Kyowa Hakko DC-102, Kyowa Hakko DC-79, Kyowa Hakko DC-88A, Kyowa Hakko DC89-A1, Kyowa Hakko DC92-B, Ditrisarubicin B, Shionogi DOB-41, Doxorubicin, Doxorubicin-Fibrinogen, Elsamicin A, Epirubicin, Erbstatin, Esorubicin, Esperamicin-A1, Esperamicin-Alb, Erbamont FCE-21954, Fujisawa FK-973, Fostriecin, Fujisawa FR-900482, Glidobactin, Gregatin-A, Grincamycin, Herbimycin, ldarubicin, Illudinen, Kazusamycin, Kesarirhodinen, Kyowa Hakko KM-5539, Kirin Brewery KRN-8602,KyowaHakkoKT-5432,KyowaHakkoKT-5594,KyowaHakkoKT-6149,AmericanCyanamid LL-D49194, Meiji Seika ME 2303, Menogaril, Mitomycin, Mitoxantron, SmithKline M-TAG, Neoenactin, Nippon Kayaku NK-313, Nippon Kayaku NKT-01, SRI International NSC-357704, Oxalysin, Oxaunomycin, Peplomycin, Pilatin, Pirarubicin, Porothramycin, Pyrindamycin A, Tobishi RA-I, Rapamycin, Rhizoxin, Rodorubicin, Sibanomicin, Siwenmycin, Sumitomo SM-5887, Snow Brand SN-706,SnowBrandSN-07,Sorangicin-A,Spasomycin,SSPharmaceuticalSS-21020,SSPharmaceutical SS-7313B, SS Pharmaceutical SS-9816B, Steffimycin B, Taiho 4181-2, Talisomycin, Takeda TAN-868A, Terpentecin, Thrazin, Tricrozarin A, Upjohn U-73975, Kyowa Hakko UCN-10028A, Fujisawa WF-3405, Yoshitomi Y-25024 und Zorubicin bestehenden Gruppe ausgewählt ist.

11. Verwendung nach Anspruch 10, worin das Antineoplastikum vom Typ Antibiotikum eine Anthrachinon-Verbindung ist.

12. Verwendung nach Anspruch 11, worin die Anthrachinon-Verbindung eine Anthracyclin-Verbindung ist.

13. Verwendung nach Anspruch 12, worin die Anthracyclin-Verbindung aus der aus Aclarubicin, Daunorubicin, Ditrisarubicin, Doxorubicin, Epirubicin, Esorubicin, Idarubicin, Pirarubicin, Rodorubicin und Zorubicin bestehenden Gruppe ausgewählt ist.

14. Verwendung nach Anspruch 12, worin die Anthracyclin-Verbindung durch ein Bakterium der Gattung Streptomycetes produziert wird.

15. Verwendung nach Anspruch 12, worin das Anthracyclin die Formel 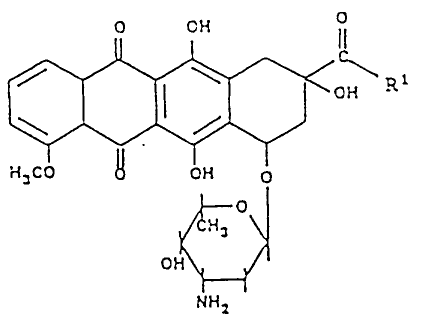 hat, worin R¹ aus geradkettigem oder verzweigtem C₁-C₅-Alkyl und C₁-C₅-Hydroxyalkyl ausgewählt ist.

16. Verwendung nach Anspruch 15, worin R¹ aus Methyl, Ethyl, n-Propyl, Isopropyl, n-Butyl, Isobutyl, sec-Butyl, tert-Butyl, n-Pentyl, Isopentyl, Neopentyl, Hydroxymethyl, Hydroxyethyl, Hydroxypropyl, Hydroxybutyl und Hydroxypentyl ausgewählt ist.

17. Verwendung nach Anspruch 16, worin die Anthracyclin-Verbindung Daunorubicin ist.

18. Verwendung nach Anspruch 17, worin die Anthracyclin-Verbindung Doxorubicin ist.

19. Verwendung nach Anspruch 1, worin das direkt wirkende Mittel aus der aus alpha-Caroten, alpha-Difluormethylarginin, Acitretin, Biotec AD-5, Kyorin AHC-52, Alstonin, Amonafid, Amphethinil, Amsacrin, Angiostat, Ankinomycin, Antineoplaston A10, Antineoplaston A2, Antineoplaston A3, Antineoplaston A5, Antineoplaston AS2-1, Henkel APD, Aphidicolinglycinat, Asparaginase, Avarol, Baccharin, Batracyclin, Benfluron, Benzotript, Ipsen-Beaufour BIM-23015, Bisantren, Bristol-Myers BMY-40481, Vestar Boron-10, Bromofosfamid, Wellcome BW-502, Wellcome BW-773, Caracemid, Carmethizol-hydrochlorid, Ajinomoto CDAF, Chlorsulfachinoxalon, Chemes CHX-2053, Chemex CHX-100, Warner-Lambert CI-921, Warner-Lambert CI-937, Warner-Lambert CI-941, Warner-Lambert CI-958, Clanfenur, Claviridenon, ICN Verbindung 1259, ICN Verbindung 4711, Contracan, Yakult Honsha CPT-11, Crisnatol, Curaderm, Cytochalasin B, Cytarabin, Cytocytin, Merz D-609, DABIS Maleat, Dacarbazin, Datelliptinium, Didemnin-B, Dihämatoporphyrinether, Dihydrolenperon, Dinalin, Distamycin, Toyo Pharmar DM-341, Toyo Pharmar DM-75, Daiichi Seiyaku DN-9693, Elliprabin, Elliptiniumacetat, Tsumura EPMTC, Ergotamin, Etoposid, Etretinat, Fenretinid, Fujisawa FR-57704, Galliumnitrat, Genkwadaphnin, Chugai GLA-43, Glaxo GR-63178, Grifolan NMF-5N, Hexadecylphosphocholin, Green Cross HO-221, Homoharringtonin, Hydroxyharnstoff, BTG ICRF-187, Ilmofosin, Isoglutamin, Isotretinoin, Otsuka JI-36, Ramot K-477,OtsuakK-76COONa,KurehaChemicalK-AM,MECTCorp.KI-8110,AmericanCyanamidL-623, Leukoregulin, Lonidamin, Lundbeck LU-23-112, Lilly LY-186641, NCI (US) MAP, Marycin, Merrel DowMDL-27048,MDL-27048,MedcoMEDR-340,Merbaron,Merocyanin-Derivaten,Methylanilinoacridin, Molecular Genetics MGI-136, Minactivin, Mitonafid, Mitoquidon, Mitotan, Mopidamol, Motretinid, Zenyaku Kogyo MST-16, N-(Retinoyl)aminosäuren, Nisshin Flour Milling N-021, N-acelierten Dehydroalaninen, Nafazatrom, Taisho NCU-190, Nocodazol-Derivat, Normosang, NCI NSC-145813, NCI NSC-361456, NCI NSC-604782, NCI NSC-95580, Octreotid, Ono ONO-112, Oquizanocin, Akzo Org-10172, Pancratistatin, Pazelliptin, Warner-Lambert PD-111707, Warner-Lambert PD-115934, Warner-Lambert PD-131141, Pierre Fabre RE-1001, ICRT Peptid D, Piroxantron, Polyhämatoporphyrin, polypreic-Säure, Efamol-Porphyrin, Probiman, Procarbazin, Proglumid, Invitron Protease-Nexin I, Tobishi RA-700, Razoxan, Sapporo Breweries RBS, Restrictin-P, Retelliptin, Retinsäure, Rhone-Roulenc RP-49532, Rhone-Poulenc RP-56976, SmithKline SK&F-104864, Sumitomo SM-108, Kuraray SMANCS, SeaPharm SP-10094, Spatol, Spirocyclopropan-Derivaten, Spirogermanium, Unimed, SS Pharmaceutical SS-554, Strypoldinon, Stypoldion, Suntory SUN 0237, Suntory SUN 2071, Superoxid-Dismutase, Toyama T-506, Toyama T-680, Taxol, Teijin TEI-0303, Teniposid, Thaliblastin, Eastman Kodak TJB-29, Tocotrienol, Topostin, Teijin TT-82,KyowaHakkoUCN-01,KyowaHakkoUCN-1028,Ukrain,EastmanKodakUSB-006,Vinblastinsulfat, Vincristin, Vindesin, Vinestramid und Vinorelbin, Vintriptol, Vinzolidin, Withanoliden, Yamanouchi YM-534 bestehenden Gruppe ausgewählt ist.

20. Verwendung nach Anspruch 1, worin das cytoprotektive Copolymer Carbetimer ist und das direkt wirkende Mittel ein Antineoplastikum vom Typ Anthracyclin ist.

21. Verwendung nach Anspruch 20, worin das Antineoplastikum vom Typ Anthracyclin aus der aus Aclarubicin, Daunorubicin, Ditrisarubicin, Doxorubicin, Epirubicin, Esorubicin, Idarubicin, Pirarubicin, Rodorubicin und Zorubicin bestehenden Gruppe ausgewählt ist.

22. Verwendung nach Anspruch 21, worin das Antineoplastikum vom Typ Anthracyclin Doxorubicin ist.

23. Verwendung nach Anspruch 1, worin das cytoprotektive Copolymer Carbetimer ist.

24. Verwendung nach Anspruch 23 zur Herstellung eines Arzneimittels zur Behandlung akuter Leukämien, maligner Lymphome, der Hodgkin-Krankheit, von Ovarialkarzinom, Brustkrebs, Kleinzellenkarzinom der Lunge, osteogenem Karzinom, Ewing-Karzinom, Weichteilkarzinomen, metastatischem Brustadenokarzinom, Blasenkrebs, bronchogenem Karzinom, Neuroblastom, metastatischem Schilddrüsenkarzinom, Endometriumkarzinom, Hodenkrebs, Prostatakarzinom, Zervixkarzinom, Karzinomen im Kopf- und Halsbereich, Magenkarzinom und Plasmazellenmyelom.

25. Verwendung nach Anspruch 24, worin das direkt wirkende Mittel ein Antineoplastikum vom Typ Anthracyclin ist.

26. Verwendung nach Anspruch 25, worin das Antineoplastikum vom Typ Anthracyclin aus der aus Aclarubicin, Daunorubicin, Ditrisarubicin, Doxorubicin, Epirubicin, Esorubicin, Idarubicin, Pirarubicin, Rodorubicin und Zorubicin bestehenden Gruppe ausgewählt ist.

## Revendications (Revendications pour l'(les) Etat(s) contractant(s) suivant(s): AT, BE, DE, DK, FR, GB, IT, LU, NL, SE, CH)

1. Une combinaison comprenant un copolymère cytoprotecteur et un ou plusieurs agents antinéoplasiques du type antimétabolite à action directe, selon laquelle ledit copolymère cytoprotecteur est un copolymère semi-amide:semi-imide comprenant des unités monomères de
(a) un groupe semi-amide, semi-carboxyle de formule : 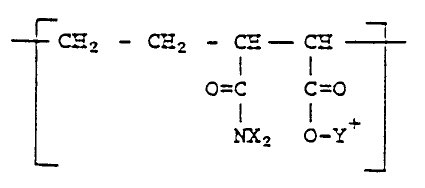 et
(b) d'imide de formule : 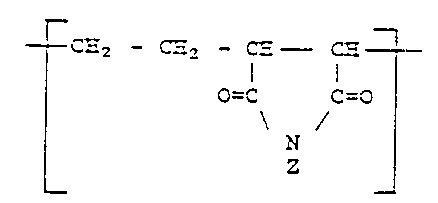
dans laquelle X est choisi indépendamment parmi les groupes hydrido et C₁-C₄-alkyle ; dans laquelle Y est choisi parmi les groupes hydrido, ammonium et les cations métalliques pharmaceutiquement acceptables ; et dans laquelle Z est choisi parmi les groupes hydrido, C₁-C₄-alkyle, ammonium et les cations métalliques pharmaceutiquement acceptables.

2. La combinaison selon la revendication 1, selon laquelle ledit copolymère semi-amide:semi-imide cytoprotectcur est un copolymère d'au moins un monomère d'oléfine ayant de 2 à 4 atomes de carbone et d'au moins un anhydride polycarboxylique α,β-insaturé ayant de 4 à 6 atomes de carbone, ayant un poids moléculaire moyen de 300 à 1 800 et dérivatisé pour obtenir à la fois (a) des groupes semi-amides, semi-acides carboxyliques et (b) des groupes imides où lesdits groupes imides comprennent de 5 à 40 % en poids desdits groupes dérivatisés, et les dérivés N-alkylés et les dérivés de sels cationiques pharmaceutiquement acceptables dudit copelymère dérivatisé, lesdits dérivés N-alkylés ayant de 1 à 4 atomes de carbone dans les substituants alkyles.

3. La combinaison selon la revendication 2, selon laquelle ledit copolymère semi-amide:semi-imide cytoprotecteur est un copolymère d'éthylène et d'anhydride maléique comprenant :
(a) un sel semi-amide, semi-ammonium de formule : 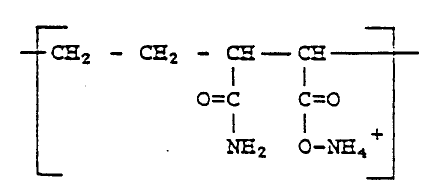 et
(b) un imide non substitué de formule :

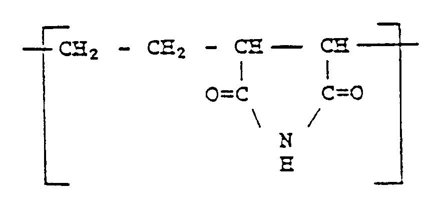

4. La combinaison selon la revendication 3, selon laquelle ledit copolymère semi-amide:semi-imide est de formule : 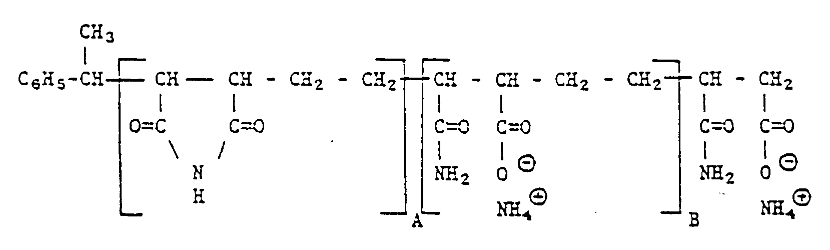 dans laquelle le rapport entre A et B est dans l'intervalle de 1:2 à 1:5.

5. La combinaison selon la revendication 4, selon laquelle ledit copolymère semi-amide:semi-imide est le carbetimer.

6. La combinaison selon la revendication 1, selon laquelle l'agent antinéoplasique antimétabolite est choisi dans le groupe comprenant les suivants : 5-FU-fibrinogène, acide acanthifolique, aminothiadiazole, brequinar sodium, carmofur, Ciba-Geigy CGP-30694, cyclopentylcytoxine, cytarabine phosphate stéarate, conjugués de cytarabine, Lilly DATHF, Merrel Dow DDFC, dézaguanine, didéoxycytidine, didéoxyguanosine, didox, Yoshitomi DMDC, doxifluridine, Welcome EHNA, Merck & Co. EX-015, fazarabine, floxuridine, fludarabine phosphate, 5-fluorouracile, N-(2'-furanidyl)-5-fluorouracile, Daiichi Seiyaku FO-152, isopropylpyrrolizine, Lilly LY-188011, Lilly LY264618, méthobenzaprime, méthotrexate, Wellcome MSPES, norspermidine, NCI NSC-127716, NCI NSC-264880, NCI NSC-39661, NCI NSC-612567, Warner-Lambert PALA, pentostatine, piritrexime, plicamycine, Asahi Chemical PL-AC, Takeda TAC-788, thioguanine, tiazofurine, Erbamont TIF, trimétrexate, inhibiteurs de tyrosine-kinase, inhibiteurs de protéine-kinase, Taiho UFT et uricytine.

7. La combinaison selon la revendication 1, selon laquelle ledit agent à action directe est un agent antinéoplasique à action alkylante.

8. La combinaison selon la revendieation 7, selon laquelle ledit agent antinéoplasique du type alkylant est choisi dans le groupe comprenant les suivants:Shionogi 254-S, analogues aldo-phosphamides, altrétamine, anaxirone, Boehringer Mannheim BBR-2207, bestrabucile, budotitane, Wakunaga CA-102, carboplatine, carmustine, Chinoin-139, Chinoin-153, chlorambucile, cisplatine, cyclophosphamide, American Cyanamid CL-286558, Sanofi CY-233, cyplatate, Degussa D-19-384, Sumimoto DACHP(Pyr)2, diphénylspiromustine, diplatine cytostatique, dérivés de Erba distamycine, Chugai DWA-2114R, ITI E09, elmustine, Erbamont FCE-24517, estramustine phosphate sodium, fotémustine, Unimed G-6-M, Chinoin GYKI-17230, heptsulfame, ifosfamide, iproplatine, lomustine, mafosfamide, mitolactol, Nippon Kayaku NK-121, NCI NSC-264395, NCI NSC-342215, oxaliplatine, Upjohn PCNU, prednimustine, Proter PTT-119, ranimustine, sémustine, Smithkline SK&F-101772, Yakult Honsha SN-22, spiromustine, Tanabe Seiyaku TA-077, tauromustine, témozolomide, téroxirone, tétraplatine et trimélamol.

9. La combinaison selon la revendication 1, selon laquelle ledit agent à action directe est un agent antinéoplasique du type antibiotique.

10. La combinaison selon la revendication 8, selon laquelle ledit agent antinéoplasique du type antibiotique est choisi dans le groupe comprenant les composés suivants : Taiho 4181-A, aclarubicine, actinomycine D, actinoplanone, Erbamont ADR-456, dérivé d'aéroplysinine, Ajinomoto AN-201-II, Ajinomoto AN-3, Nipon Soda anisomycines, anthracycline, azino-mycine-A,bisucabérine, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, sulfate de bléomycine, bryostatine-1, Taiho C-1027, calichemycine, chromoximycine, dactinomycine, daunorubicine, Kyowa Hakko DC-102, Kyowa Hakko DC-79, Kyowa Hakko DC-88A, Kyowa Hakko DC89-A1, Kyowa Hakko DC92-B, ditrisarubicine B, shionogi DOB-41, doxorubicine, doxorubicine-fibrinogène, elsamicine-A, épirubicine, erbstatine, esorubicine, espéramicine-A1, espéramicine-A1b, Erbamont FCE-21954, Fujisawa FK-973, fostriécine, Fujisawa FR-900482, glidobactine, grégatine A, grincamycine, herbimycine, idarubicine, illudines, kazusamycine, késarirhodines, Kyowa Hakko KM-5539, Kirin Brewery DRN-8602, Kyowa Hakko KT-5432, Kyowa Hakko KT-5594, Kyowa Hakko KT-6149, American Cyanamid LL-D49194, Meiji Seika ME 2303, menogaril, mytomycine, mitoxantrone, SmithKline M-TAG, néoénactine, Nippon Kayaku NK-313, Nippon Kayaku NKT-01, SRI International NSC-357704, oxalysine, oxaunomycine, peplomycine, pilatine, pyrarubicine, perothramycine, pyrindamycine A, Tobishi RA-I, rapamycine, rhizoxine, rodorubicine, sibanomicine, siwenmycine, Sumito SM-5887, Snow Brand SN-707, Snow Brand SN-07, sorangicine-A, spaffomycine, SS Pharmaceutical SS-21020, SS Pharmaccutical SS-7313B, SS Pharmaceutical SS-9816B, steffimycine B, Taiho 4181-2, talisomycine, Takeda TAN-868A, terpentécine, thrazine, tricrozarine A, Upjohn U-74975, Kyowa Hakko UCN-10028A, Fujisawa WF-3405, Yoshitomo Y-25024 et zorubicine.

11. La combinaison selon la revendication 10, selon laquelle ledit agent antinéoplasique du type antibiotique est un composé d'anthraquinone.

12. La combinaison selon la revendication 11, selon laquelle ledit composé d'anthraquinone est un composé d'anthracycline.

13. La combinaison selon la revendication 12, selon laquelle ledit composé d'anthracycline est choisi dans le groupe comprenant les suivants : aclatubicine, daunorubicine, ditrisarubicine, doxorubicine, epirubicine, esorubicine, idarubicine, pirarubicine, rodorubicine et zorubicine.

14. La combinaison selon la revendication 12, selon laquelle ledit composé d'anthracycline est produit par une bactérie du genre Streptomyctes.

15. La combinaison selon la revendication 12, selon laquelle ledit composé d'anthracycline a la formule : 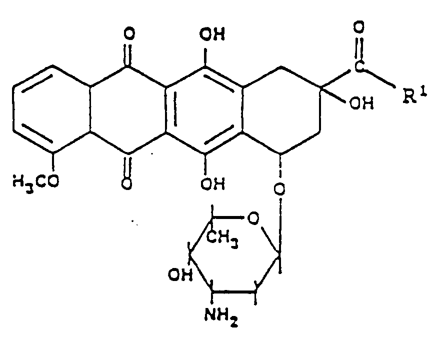 dans laquelle R¹ est choisi parmi les groupes C₁-C₅-alkyles et C₁-C₅-hydroxy-alkyles à chaîne droite ou ramifiée.

16. La combinaison selon la revendication 15, selon laquelle R¹ est choisi parmi les suivants : méthyle, éthyle, n-propyle, isopropyle, n-butyle, isobutyle, sec-butyle, tert-butyle, n-pentyle, iso-pentyle, néopentyle, hydroxy-méthyle, hydroxyéthyle, hydroxypropyle, hydroxybutyle et hydroxypentyle.

17. La combinaison selon la revendication 16, selon laquelle ledit composé d'anthracycline est la daunorubicine.

18. La combinaison selon la revendication 16, selon laquelle ledit composé d'anthracycline est la doxorubicine.

19. La combinaison selon la revendication 1, selon laquelle l'agent actif est choisi dans le groupe comprenant les suivants : alpha-carotène, alpha-difluorométhyl-arginine, acitretine, Biotec AD-5, Kyorin AHC-52, alstonine, amonafide, amphéthinile, amsacrine, Angiostat, ankinomycine, antinéoplaston A19, antinéoplaston A2, antinéoplaston A3, antinéoplaston A5, antinéoplaston AS2-1, Henkel APD, aphidicoline glycinate, asparaginase, Avarol, baccharine, batracyline, benfluron, beiizotript, Ipsen-Beaufour BIM-23015, bisantrène, Bristo-Myers BMY-40481, Vestar boron-10, bromofosfamide, Wellcome BW-502, Wellcome BW-773, caracémide, carméthizole chlorhydrate, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfénur, claviridénone, ICN composé 1259, ICN composé 4711,Contracan, Yakult Honsha CPT-11, crisnatol, Curaderme, cytochalasine B, cytarabine, cytocytine, Merz D-609, DABIS maléate, dacarbazine, datelliptinium, didemnine-B, éther de dihaématoporphyrine, dihydrolenpérone, dinaline, distamycine, Toyo Pharmar DM-341, Toyo Pharmar DM-75, Daiichi Seiyaku DN-9493, elliprabine, elliptinium acétate, Tsumura EPMTC, ergotamine, étoposide étretinate, fenrétinide, Fujisawa FR-57704, gallium nitrate, genkwadaphnine, Chugai, GLA-43, Glaxo GR-63178, grifolane NMF-5N, hexadécylphosphocholine, Green Cross HO-221, homoharringtonine, hydroxyarea, BTG ICRF-187,ilmofosine, isoglutamine, isotretinoine, Otsuka JI-36, Ramot K-477, Otsuak K-76COONa, Kureha Chemical K-AM, MECT Corps. KI-8110, American Cyanamid L-623, leukoreguline, lonidamine, Lundbeck LU-23-112, Lilly LY-186641,NCI (US) MAP, marycine, Merrel Dow MDL-27048, MDL-27048, Medco MEDR-340, merbarone, dérivés de mérocyanine, méthylanilinoacridine, Molecular Genetics MGI-136, minactivine, mitonafide, mitoquidone, mitotane, mopidamol, motretinide, Zenyaku Kygyo MST-16, N-(rétinoyl)amino acidcs, Nisshin Flour Milling N-021, déhydroalanines N-acylées, nafazatrom, Taisho NCU-190, dérivé de nocodazole, Normosang, NCI NSC-145813, NCI-NSC-361456, NCI NSC-604782, NCI NSC-95580, octréotide, Ono ONO-112, oquizanocine, Akzo Org-10172, pancratistatine, pazelliptine, Warner-Lambert Pd-111707, Warner-Lambert PD-115934, Warner-Lambert PD-131141, Pierre Fabre PE-1001, ICRT peptide D, Pyroxantrone, polyhaématoporphyrine, acice polypréique, Efamol porphyrine, probimane, procarbazine, proglumide, Invitron protéase nexine I, Tobishi RA-700, razoxane, Sapporo Breweries RBS, restrictine-P, retelliptine, acide rétinoïque, Rhone-Poulenc RP-49532, Rhone-Poulenc RP-56976, SmithKline SK&F-104864, Sumitomo SM-108, Kuraray SMANCS, SeaPharm SP-10094, spatol, dérivés de spirocyclopropane, siprogermanium, Unimed, SS Pharmaceutical SS-554, strypoldinone, Stypoldione, Suntory SUN 0237, Suntory SUN 2071, superoxyde dismutase, Toyama T-506, Toyama T-680, taxol, Teijin TEI-0303, teniposide, thaliblastine, Eastman Kodak TJB-29, tocotriénol, Topostine, Teijin TT-82, Kyowa Hakko UNC-01, Kyowa Hakko UNC-1028, ukraine, Eastman Kodak USB-006, vinblastine sulfate, vincristine, vindésine, vinestramide et vinorelbine, vintriptol, vinzolidine, withanolides, Yamanouchi YM-534.

20. Une combinaison selon la revendication 1, selon laquelle ledit copolymère cytoprotecteur est le carbetimer et selon laquelle l'agent à action directe est un agent antinéoplasique du type anthracycline.

21. La combinaison selon la revendication 20, selon laquelle ledit agent antinéoplasique du type anthracycline est choisi dans le groupe comprenant les suivants : aclarubicine, daunorubicine, ditrisarubicine, doxorubicine, épirubicine, ésorubicine, idarubicine, pirarubicine, rodorubicine et zorubicine.

22. La combinaison selon la revendication 21, selon laquelle ledit agent antinéoplasique du type anthracycline est la doxorubicine.

23. Utilisation d'un agent antinéoplasique et du carbetimer pour la préparation d'un médicament pour le traitement de la néoplasie chez un patient.

24. Utilisation selon la revendication 23 pour la préparation d'un médicament pour le traitement des leucémies aiguës, des lymphomes malignes, de la maladie de Hodgkin, du carcinome ovarien, du carcinome du sein, du carcinome des petites cellules du poumon, du carcinome ostéogène, du carcinome de Ewing, du carcinome des tissus mous, de l'adérocarcinome du sein métastatique, du carcinome de la vessie, du carcinome bronchogène, de la neuroblastome, du carcinome de la thyroïde métastatique, du carcinome de l'endomètre, du carcinome du testicule, du carcinome de la prostate, du col de l'utérus, du carcinome de la tête et du cou, du carcinome gastrique et du myélome de la cellule plasmatique.

25. Utilisation selon la revendication 24, selon laquelle ledit agent antinéoplasique est un agent antinéoplasique du type anthracycline.

26. Utilisation selon la revendication 25, selon laquelle ledit agent antinéoplasique du type anthracycline est choisi dans le groupe comprenant les suivants : aclarubicine, daunorubicine, ditrisarubicine, doxorubicine, epirubicine, ésorubicine, idarubicine, piratubicine, rodorubicine et zorubicine.

27. Utilisation selon la revendication 26, selon laquelle ledit agent antinéoplasique du type anthracycline est la doxorubicine.

## Revendications (Revendications pour l'(les) Etat(s) contractant(s) suivant(s): ES, GR)

1. Utilisation d'un copolymère cytoprotecteur et d'un ou de plusieurs agents antinéoplasiques du type antimétabolite à action directe pour la préparation d'un médicament pour le traitement de la néoplasie chez un patient, selon laquelle ledit copolymère cytoprotecteur est un copolymère semi-amide:semi-imide comprenant des unités monomères de
(a) un groupe semi-amide,semi-carboxyle de formule : 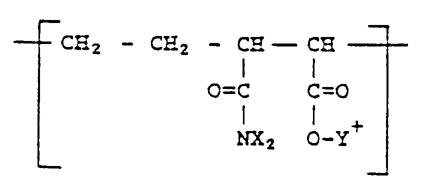 et
(b) d'imide de formule : 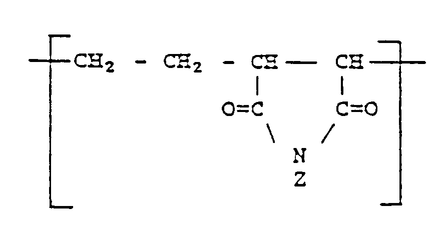
dans laquelle X est choisi indépendamment parmi les groupes hydrido et C₁-C₄-alkyle ; dans laquelle Y est choisi parmi les groupes hydrido, ammonium et les cations métalliques pharmaceutiquement acceptables ; et dans laquelle Z est choisi parmi les groupes hydrido, C₁-C₄-alkyle, ammonium et les cations métalliques pharmaceutiquement acceptables.

2. Utilisation selon la revendication 1, selon laquelle ledit copolymère semi-amide:semi-imide cytoprotecteur est un copolymère d'au moins un monomère oléfinique ayant de 2 à 4 atomes de carbone et d'au moins un anhydride polycarboxylique α,β-insaturé ayant de 4 à 6 atomes de carbone, ayant un poids moléculaire moyen de 300 à 1 800 et dérivatisé pour obtenir à la fois (a) des groupes semi-amides, semi-acides carboxyliques et (b) des groupes imides où lesdits groupes imides comprennent de 5 à 40 % en poids desdits groupes dérivatisés, et les dérivés N-alkylés et les dérivés de sels cationiques pharmaceutiquement acceptables dudit copolymère dérivatisé, lesdits dérivés N-alkylés ayant de 1 à 4 atomes de carbone dans les substituants alkyles.

3. Utilisation selon la revendication 2, selon laquelle ledit copolymère semi-amide:semi-imide cytoprotecteur est un copolymère d'éthylène et d'anhydride maléique comprenant :
(a) un sel semi-amide, semi-ammonium de formule : 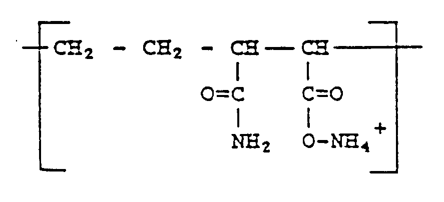 et
(b) un imide non substitué de formule :

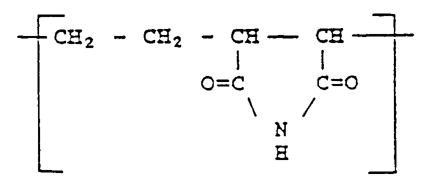

4. Utilisation selon la revendication 3, selon laquelle ledit copolymère semi-amide:semi-imide a la formule : 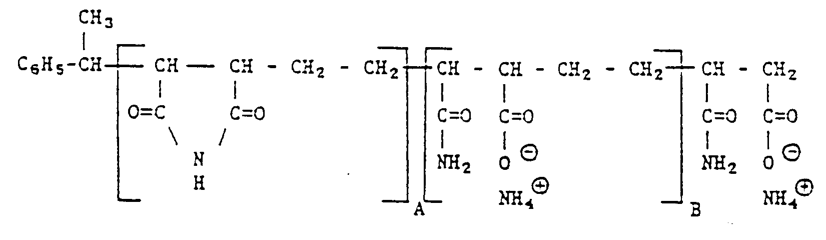 dans laquelle le rapport entre A et B est dans l'intervalle de 1:2 à 1:5.

5. Utilisation selon la revendication 4, selon laquelle ledit copolymère semi-amide:semi-imide est le carbetimer.

6. Utilisation selon la revendication 1, selon laquelle l'agent antinéoplasique antimétabolite est choisi dans le groupe constitué de 5-FU-fibrinogène, acide acanthifolique, aminothiadiazole, brequinar sodium, carmofur, Ciba-Geigy CGP-30694, cyclopentylcytoxine, cytarabine phosphate stéarate, conjugués de cytarabine, Lilly DATHF, Merrel Dow DDFC, dézaguanine, didéoxycytidine, didéoxyguanosine, didox, Yoshitomi DMDC, doxifluridine, Welcome EHNA, Merck & Co. EX-015, fazarabine, floxuridine, fludarabine phosphate, 5-fluorouracile, N-(2'-furanidyl)-5-fluorouracile, Daiichi Seiyaku FO-152, isopropylpyrrolizine, Lilly LY-188011, Lilly LY-264618, méthobenzaprime, méthotrexate, Wellcome MSPES, norspermidine, NCI NSC-127716, NCI NSC-264880, NCI NSC-39661, NCI NSC-612567, Warner-Lambert PALA, pentostatine, piritrexime, plicamycine, Asahi Chemical PL-AC, Takeda TAC-788, thioguanine, tiazofurine, Erbamont TIF, trimétrexate, inhibiteurs de tyrosine-kinase, inhibiteurs de protéine-kinase, Taiho UFT et uricytine.

7. Utilisation selon la revendication 1, selon laquelle ledit agent à action directe est un agent antinéoplasique du type alkylant.

8. Utilisation selon la revendication 7, selon laquelle l'agent antinéoplasique du type alkylant est choisi dans le groupe constitué de composés suivants : Shionogi 254-S, analogues aldo-phosphamides, altrétamine, anaxirone, Boehringer Mannheim BBR-2207, bestrabucile, budotitane, Wakunaga CA-102, carboplatine, carmustine, Chinoin-139, Chinoin-153, chlorambucile, cisplatine, cyclophosphamide, American Cyanamid CL-286558, Sanofi CY-233, cyplatate, Degussa D-19-384, Sumimoto DACHP(Pyr)2, diphénylspiromustine, diplatine cytostatique, dérivés de Erba distamycine, Chugai DWA-2114R, ITI E09, elmustine, Erbamont FCE-24517, estramustine phosphate sodium, fotémustine, Unimed G-6-M, Chinoin GYKI-17230, heptsulfame, ifosfamide, iproplatine, lomustine, mafosfamide, mitolactol, Nippon Kayaku NK-121, NCI NSC-264395, NCI NSC-342215, oxaliplatine, Upjohn PCNU, prednimustine, Proter PTT-119, ranimustine, sémustine, Smithkline SK&F-101772, Yakult Honsha SN-22, spiromustine, Tanabe Seiyaku TA-077, tauromustine, témozolomide, téroxirone, tétraplatine et trimélamol.

9. Utilisation selon la revendication 1, selon laquelle ledit agent à action directe est un agent antinéoplasique du type antibiotique.

10. Utilisation selon la revendication 9, selon laquelle ledit agent antinéoplasique du type antibiotique est choisi dans le groupe comprenant les composés suivants : Taiho 4181-A, aclarubicine, actinomycine D, actinoplanone, Erbamont ADR-456, dérivé d'aéroplysinine, Ajinomoto AN-201-II, Ajinomoto AN-3, Nipon Soda anisomycines, anthracycline, azino-mycinc-A,bisucabérine, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, sulfate de bléomycine, bryostatine-1, Taiho C-1027, calichemycine, chromoximycine, dactinomycine, daunorubicine, Kyowa Hakko DC-102, Kyowa Hakko DC-79, Kyowa Hakko DC-88A, Kyowa Hakko DC89-A1, Kyowa Hakko DC92-B, ditrisarubicine B, shionogi DOB-41, doxorubicine, doxorubicine-fibrinogène, elsamicine-A, épirubicine, erbstatine, esorubicine, espéramicine-A1, espéramicine-A1b, Erbamont FCE-21954, Fujisawa FK-973, fostriécine, Fujisawa ER-900482, glidobactine, grégatine A, grincamycine, herbimycine, idarubicine, illudines, kazusamycine, késarirhodines, Kyowa Hakko KM-5539, Kirin Brewery DRN-8602, Kyowa Hakko KT-5432, Kyowa Hakko KT-5594, Kyowa Hakko KT-6149, American Cyanamid LL-D49194, Meiji Seika ME 2303, menogaril, mytomycine, mitoxantrone, SmithKline M-TAG, néoénactine, Nippon Kayaku NK-313, Nippon Kayaku NKT-01, SRI International NSC-357704, oxalysine, oxaunomycine, peplomycine, pilatine, pyrarubicine, porothramycine, pyrindamycine A, Tobishi RA-I, rapamycine, rhizoxine, rodorubicine, sibanomicinc, siwenmycine, Sumito SM-5887, Snow Brand SN-707, Snow Brand SN-07, sorangicine-A, sparsomycine, SS Pharmaceutical SS-21020, SS Pharmaceutical SS-7313B, SS Pharmaceutical SS-9816B, steffimycine B, Taiho 4181-2, talisomycine, Takeda TAN-868A, terpentécine, thrazine, tricrozarine A, Upjohn U-74975, Kyowa Hakko UCN-10028A, Fujisawa WF-3405, Yoshitomo Y-25024 et zorubicine.

11. Utilisation selon la revendication 10, selon laquelle ledit agent antinéoplasique du type antibiotique est un composé d'anthraquinone.

12. Utilisation selon la revendication 11, selon laquelle le composé d'anthraquinone est un composé d'anthracycline.

13. Utilisation selon la revendication 12, selon laquelle ledit composé d'anthracycline est choisi dans le groupe comprenant les composés suivants : aclarubicine, daunorubicine, ditrisarubicine, doxorubicine, epirubicine, esorubicine, idarubicine, pirarubicine, rodorubicine et zorubicine.

14. Utilisation selon la revendication 12, selon laquelle le composé d'anthracycline est produit par une bactérie du genre Streptomycetes.

15. Utilisation selon la revendication 12, selon laquelle ladite anthracycline a la formule : 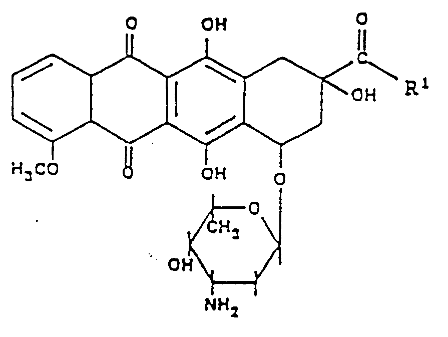 dans laquelle R¹ est choisi parmi les groupes C₁-C₅-alkyles et C₁-C₅-hydroxy-alkyles à chaîne droite ou ramifiée.

16. Utilisation selon la revendication 15, selon laquelle R¹ est choisi parmi les suivants : méthyle, éthyle, n-propyle, isopropyle, n-butyle, isobutyle, sec-butyle, tert-butyle, n-pentyle, iso-pentyle, néopentyle, hydroxyméthyle, hydroxyéthyle, hydroxypropyle, hydroxybutyle et hydroxypentyle.

17. Utilisation selon la revendication 16, selon laquelle ledit composé d'anthracycline est la daunorubicine.

18. Utilisation selon la revendication 17, selon laquelle ledit composé d'anthracycline est la doxorubicine.

19. Utilisation selon la revendication 1, selon laquelle ledit agent à action est choisi dans le groupe comprenant les composés suivants : alpha-carotène, alpha-difluorométhyl-arginine, acitretine, Biotec AD-5, Kyorin AHC-52, alstonine, amonafide, amphéthinile, amsacrine, Angiostat, ankinomycine, antinéoplaston A19, antinéoplaston A2, antinéoplaston A3, antinéoplaston A5, antinéoplaston AS2-1, Henkel APD, aphidicoline glycinate, asparaginase, Avarol, baccharine, batracyline, benfluron, benzotript, Ipsen-Beaufour BIM-23015, bisantrène, Bristo-Myers BMY-40481, Vestar boron-10, bromofosfamide, Wellcome BW-502, Wellcome BW-773, caracémide, carméthizole chlorhydrate, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfénur, claviridénone, ICN composé 1259, ICN composé 4711,Contracan, Yakult Honsha CPT-11, crisnatol, Curaderme, cytochalasine B, cytarabine, cytocytine, Merz D-609, DABIS maléate, dacarbazine, datelliptinium, didemnine-B, éther de dihaématoporphyrine, dihydrolenpérone, dinaline, distamycine, Toyo Pharmar DM-341, Toyo Pharmar DM-75, Daiichi Seiyaku DN-9493, elliprabine, elliptinium acétate, Tsumura EPMTC, ergotamine, étoposide étretinate, fenrétinide, Fujisawa FR-57704, gallium nitrate, genkwadaphnine, Chugai, GLA-43, Glaxo GR-63178, grifolane NMF-5N, hexadécylphosphocholine, Green Cross HO-221, homoharringtonine, hydroxyarea, BTG ICRF-187, ilmofosine, isoglutamine, isotretinoine, Otsuka JI-36, Ramot K-477, Otsuak K-76COONa, Kureha Chemical K-AM, MECT Corps. KI-8110, American Cyanamid L-623, leukoreguline, lonidamine, Lundbeck LU-23-112, Lilly LY-186641,NCI (US) MAP, marycine, Merrel Dow MDL-27048, MDL-27048, Medco MEDR-340, merbarone, dérivés de mérocyanine, méthylanilinoacridine, Molecular Genetics MGI-136, minactivine, mitonafide, mitoquidone, mitotane, mopidamol, motretinide, Zenyaku Kygyo MST-16, N-(rétinoyl)amino acides, Nisshin Flour Milling N-021, déhydroalanines N-acylées, nafazatrom, Taisho NCU-190, dérivé de nocodazole, Normosang, NCI NSC-145813, NCI-NSC-361456, NCI NSC-604782, NCI NSC-95580, octréotide, Ono ONO-112, oquizanocine, Akzo Org-10172, pancratistatine, pazelliptine, Warner-Lambert Pd-111707, Warner-Lambert PD-115934, Warner-Lambert PD-131141, Pierre Fabre PE-1001, ICRT peptide D, Pyroxantrone, polyhaématoporphyrine, acice polypréique, Efamol porphyrine, probimane, procarbazine proglumide, Invitron protéase nexine I, Tobishi RA-700, razoxane, Sapporo Breweries RBS, restrictine-P, retelliptine, acide rétinoïque, Rhone-Poulenc RP-49532, Rhone-Poulenc RP-56976, SmithKline SK&F-104864, Sumitomo SM-108, Kuraray SMANCS, SeaPharm SP-10094, spatol, dérivés de spirocyclopropane, siprogermanium, Unimed, SS Pharmaceutical SS-554, strypoldinone, Stypoldione, Suntory SUN 0237, Suntory SUN 2071, superoxyde dismutase, Toyama T-506, Toyama T-680, taxol, Teijin TEI-0303, teniposide, thaliblastine, Eastman Kodak TJB-29, tocotriénol, Topostine, Teijin TT-82, Kyowa Hakko UNC-01, Kyowa Hakko UNC-1028, ukraine, Eastman Kodak USB-006, vinblastine sulfate, vincristine, vindésine, vinestramide et vinorelbine, vintriptol, vinzolidine, withanolides, Yamanouchi YM-534.

20. Utilisation selon la revendication 1, selon laquelle ledit copolymère cytoprotectcur est le carbetimer et l'agent à action directe est un agent antinéoplasique du type anthracycline.

21. Utilisation selon la revendication 20, selon laquelle ledit agent antinéoplasique du type anthracycline est choisi dans le groupe comprenant les composés suivants : aclarubicine, daunorubicine, ditrisarubicine, doxorubicine, épirubicine, ésorubicine, idarubicine, pirarubicine, rodorubicine et zorubicine.

22. Utilisation selon la revendication 21, selon laquelle ledit agent antinéoplasique du type anthracycline est la doxorubicine.

23. Utilisation selon la revendication selon laquelle ledit copolymère cytoprotecteur est le carbetimer.

24. Utilisation selon la revendication 23 pour la préparation d'un médicament pour le traitement des leucémies aiguës, des lymphomes malignes, de la maladie de Hodgkin, du carcinome ovarien, du carcinome du sein, du carcinome des petites cellules du poumon, du carcinome ostéogène, du carcinome de Ewing, du carcinome des tissus mous, de l'adérocarcinome du sein métastatique, du carcinome de la vessie, du carcinome bronchogène, de la neuroblastome, du carcinome de la thyroïde métastatique, du carcinome de l'endomètre, du carcinome du testicule, du carcinome de la prostate, du col de l'utétus, du carcinome de la tête et du cou, du carcinome gastrique et du myélome de la cellule plasmatique.

25. Utilisation selon la revendication 24, selon laquelle ledit agent antinéoplasique est un agent antinéoplasique du type anthracycline.

26. Utilisation selon la revendication 25, selon laquelle ledit agent antinéoplasique du type anthracycline est choisi dans le groupe comprenant les suivants : aclarubicine, daunorubicine, ditrisarubicine, doxorubicine, epirubicine, ésorubicine, idarubicine, pirarubicine, rodorubicine et zorubicine.
